# EUROPEAN PATENT APPLICATION

(11) **EP 4 272 626 A1**
(43) Date of publication of application: **08.11.2023**
(21) Application number: 21914616.4
(22) Date of filing: 30.12.2021
(51) Int. Cl.: A61B 5/00, G16H 10/60

(54) **WORKFLOW DEFINITION METHOD AND VITAL SIGN MONITORING DEVICE**

(30) Priority: 30.12.2020 WO PCT/CN2020/141349; 30.12.2020 WO PCT/CN2020/141347
(71) Applicant: Shenzhen Mindray Bio-Medical Electronics Co., Ltd., Shenzhen, Guangdong 518057 (CN)
(72) Inventor: QING, Lei, Shenzhen, Guangdong 518057 (CN); CAO, Jianfang, Shenzhen, Guangdong 518057 (CN); JIANG, Xia, Shenzhen, Guangdong 518057 (CN); HE, Zhilu, Shenzhen, Guangdong 518057 (CN)
(74) Representative: KIPA AB
(86) International application number: PCT/CN2021/143019
(87) International publication number: WO 2022/143885

(57) **Abstract**

A vital sign monitoring device, comprising a human-computer interaction apparatus (902) and a processor (903). The human-computer interaction apparatus (902) is used to display a measurement mode identifier list, a working mode definition area (200) and a workflow configuration information definition area (300); the processor (903) determines a working mode on the basis of a working mode definition instruction, the working modes comprising a continuous measurement working mode and a discontinuous measurement working mode, determines workflow configuration information on the basis of a workflow configuration information definition instruction, and determines a monitoring page on the basis of the workflow configuration information; a target measurement mode corresponding to the target workflow configuration information is selected from the measurement mode identifier list on the basis of a measurement mode selection instruction; and the human-computer interaction apparatus (902) is further used to display a target monitoring page corresponding to the target measurement mode. In the present device, a user can self-define a monitoring page on the basis of specific measurement needs, thus satisfying the user's needs for performing specialized measurement.

## Description

### CROSS-REFERENCE TO RELATED APPLICATIONS

This disclosure claims priority to PCT application No. PCT/CN2020/141349, filed on December 30, 2020, to International Patent Office, titled "WORKFLOW DEFINITION METHOD, VITAL SIGN MONITORING DEVICE AND SERVER"; and PCT application No. PCT/CN2020/141347, filed on December 30, 2020, to International Patent Office, titled "INTERFACE DISPLAY METHOD, MONITOR AND COMPUTER STORAGE MEDIUM"; the contents of both PCT applications are incorporated herein by reference in their entirety.

### TECHNICAL FIELD

The disclosure relates to the technical field of medical device, and more particularly to a workflow definition method and a vital sign monitoring device.

### BACKGROUND

In the medical field, vital sign monitoring device is a conventional device used to acquire vital sign data of patient. It is widely used in various places, such as general wards, emergency departments, outpatient clinics, and long-term care institutions outside hospital, etc., and can provide temporary or long-term vital sign monitoring for patient.

Medical staff have various requirements for the vital sign monitoring device. Although they are all used for patient vital sign measurement, the specific device workflows varies depending on the purpose and method of use of the device. At present, most of the vital sign monitoring devices only provide a general workflow that includes various preset configuration items for medical staff, but this general workflow cannot satisfies various measurement requirements.

### SUMMARY

In this regard, a vital sign monitoring device, a device management server, a workflow definition method applied to a vital sign monitoring device, and a workflow definition method applied to a device management server, are provided, aiming at configuring an applicable workflow according to different measurement requirements and improving the flexibility of monitoring devices for vital sign monitoring under different measurement requirements.

In a first aspect, an embodiment of this disclosure provides a vital sign monitoring device, including:
a human-machine interaction apparatus, which is configured to display a measurement mode identifier list, a working mode definition area, and an area for defining workflow configuration information; wherein, the measurement mode identifier list includes multiple first measurement modes and multiple second measurement modes, the measurement mode identifier list is configured to receive an measurement mode selection instruction, the working mode definition area is configured to receive a working mode definition instruction, the area for defining the workflow configuration information is configured to receive an instruction for defining the workflow configuration information; and
a processor, which is configured to:
   determine a working mode according to the working mode definition instruction, wherein the working mode includes a continuous measurement working mode and a discontinuous measurement working mode, wherein the continuous measurement working mode includes the multiple first measurement modes, and the discontinuous measurement working mode includes the multiple second measurement modes;
   determine workflow configuration information in the working mode according to the instruction for defining the workflow configuration information, and determine a monitoring page according to the workflow configuration information; and
   select a target measurement mode, which corresponds to target workflow configuration information, from the measurement mode identifier list, according to the measurement mode selection instruction;
   wherein the human-machine interaction apparatus is further configured to display a target monitoring page which corresponds to the target measurement mode.

The vital sign monitoring device provided in the embodiment of this disclosure can determine different working modes, such as continuous measurement working mode and discontinuous measurement working mode, and define monitoring pages generated by workflow configuration information under different working modes, and display a target monitoring page corresponding to a target measurement mode after the user selects the target measurement mode from the measurement mode identifier list. Accordingly, this vital sign monitoring device can customize the workflow configuration information and monitoring page for vital sign monitoring by the user, according to specific measurement requirement of the user, thus satisfying the specific monitoring requirement of the user and providing a better user experience.

In a second aspect, an embodiment of this disclosure provides a vital sign monitoring device, including:
a processor, which is configured to:
in response to a working mode definition instruction, determine a working mode, wherein the working mode includes a continuous measurement working mode and a discontinuous measurement working mode, wherein the continuous measurement working mode includes multiple first measurement modes, and the discontinuous measurement working mode includes multiple second measurement modes;
in response to an instruction for defining workflow configuration information, determine the workflow configuration information in the working mode, and determine a monitoring page according to the workflow configuration information;
in response to a measurement mode selection instruction, determine a target measurement mode among the multiple first measurement modes and the multiple second measurement modes; and
a human-machine interaction apparatus, which is configured to display a target monitoring page which corresponds to the target measurement mode.

The vital sign monitoring device provided in the embodiment of this disclosure can determine a target measurement mode through various methods, including but not limited to a selection instruction from user, determination made by device according to positioning information, determination made by device according to acquired identifier code, determination made by device according to acquired biometric information, etc., which improves the applicable performance of the device.

In a third aspect, an embodiment of this disclosure provides a vital sign monitoring device, including:
a processor, which is configured to:
acquire a target configuration index;
transmit the target configuration index to a target device which is associated with the vital sign monitoring device, wherein the target device stores corresponding relationship(s) between one or more pairs of configuration indexe(s) and workflow configuration information, and the corresponding relationship is configured to determine target workflow configuration information which corresponds to the target configuration index; and
receive the target workflow configuration information; and
a human-machine interaction apparatus, which is configured to display a target monitoring page which corresponds to the target workflow configuration information.

The vital sign monitoring device provided in the embodiment of this disclosure can acquire workflow configuration information from a third-party device, such as a target device, and display a target monitoring page to the user based on the workflow configuration information, thus improving the flexibility for defining the workflow configuration information.

In a fourth aspect, an embodiment of this disclosure provides a vital sign monitoring device, including:
a human-machine interaction apparatus, which is configured to receive an operation instruction, wherein the operation instruction is inputted by a device operator in a predetermined working mode and related to execution of a workflow; wherein the predetermined working mode includes a continuous measurement working mode or a discontinuous measurement working mode; and
a processor, which is configured to acquire workflow execution information, which is generated by the vital sign monitoring device during the execution of the workflow in the predetermined working mode based on the operation instruction; and to obtain, by self-learning the workflow execution information, workflow configuration information to be recommended, which corresponds to the predetermined working mode;
wherein the human-machine interaction apparatus is further configured to display a monitoring page to be recommended, which corresponds to the recommended workflow configuration information.

The vital sign monitoring device provided in the embodiment of this disclosure can define workflow configuration information through self-learning, and display a recommended monitoring page to the user based on the workflow configuration information for confirmation or modification, improving the configuration flexibility of the monitoring page, simplifying the manual configuration process, and providing a better user experience.

In a fifth aspect, an embodiment of this disclosure provides a workflow definition method applied to a vital sign monitoring device, which includes:
displaying a measurement mode identifier list, a working mode definition area, and an area for defining workflow configuration information; wherein the measurement mode identifier list includes multiple first measurement modes and multiple second measurement modes; the measurement mode identifier list is configured to receive an measurement mode selection instruction, the working mode definition area is configured to receive a working mode definition instruction; the area for defining the workflow configuration information is configured to receive an instruction for defining the workflow configuration information;
determining a working mode according to the working mode definition instruction; wherein the working mode includes a continuous measurement working mode and a discontinuous measurement working mode, the continuous measurement working mode includes the multiple first measurement modes, and the discontinuous measurement working mode includes the multiple second measurement modes;
determining workflow configuration information in the working mode according to the instruction for defining the workflow configuration information, and determining a monitoring page according to the workflow configuration information;
selecting a target measurement mode, which corresponds to target workflow configuration information, from the measurement mode identifier list, according to the measurement mode selection instruction; and
displaying a target monitoring page which corresponds to the target measurement mode.

In a sixth aspect, an embodiment of this disclosure provides a workflow definition method, including:
in response to a working mode definition instruction, determining a working mode, wherein the working mode includes a continuous measurement working mode and a discontinuous measurement working mode, wherein the continuous measurement working mode includes multiple first measurement modes, and the discontinuous measurement working mode includes multiple second measurement modes;
in response to an instruction for defining workflow configuration information, determining the workflow configuration information in the working mode, and determining a monitoring page according to the workflow configuration information;
in response to a measurement mode selection instruction, determining a target measurement mode among the multiple first measurement modes and the multiple second measurement modes; and
displaying a target monitoring page which corresponds to the target measurement mode.

In a seventh aspect, an embodiment of this disclosure provides a workflow definition method, including:
acquiring a target configuration index;
transmitting the target configuration index to a target device, wherein, the target device stores corresponding relationship(s) between one or more pairs of configuration indexe(s) and workflow configuration information, and the corresponding relationship is configured to determine target workflow configuration information which corresponds to the target configuration index;
receiving the target workflow configuration information; and
displaying a target monitoring page which corresponds to the target workflow configuration information.

In an eighth aspect, an embodiment of this disclosure provides a workflow definition method, including:
receiving an operation instruction, which is inputted by a device operator in a predetermined working mode and related to execution of a workflow, wherein the predetermined working mode includes a continuous measurement working mode or a discontinuous measurement working mode;
acquiring workflow execution information, which is generated by a vital sign monitoring device during the execution of the workflow in the predetermined working mode based on the operation instruction;
obtaining, by self-learning the workflow execution information, workflow configuration information to be recommended, which corresponds to the predetermined working mode; and
displaying a monitoring page to be recommended, which corresponds to the recommended workflow configuration information.

### BRIEF DESCRIPTION OF THE DRAWINGS

In order to provide a clearer explanation for embodiments of this disclosure or the technical solutions in the prior art, a brief introduction is given to the accompanying drawings required in the description of the embodiments or prior art. It is evident that the accompanying drawings in the following description are only some embodiments of this disclosure. For ordinary technical personnel in the art, other accompanying drawings can be obtained based on these drawings without any creative effort.
FIG. 1 is a flowchart of a workflow definition method.
FIG. 2A is an interface schematic diagram of a workflow definition method.
FIG. 2B is another interface schematic diagram for implementing a workflow definition method.
FIG. 3 is a schematic diagram of a receiving manner for a measurement mode selection instruction.
FIG. 4 is another flowchart of a workflow definition method.
FIG. 5 shows several implementations for acquiring a target configuration index.
FIG. 6 is another flowchart of a workflow definition method.
FIG. 7 is another flowchart of a workflow definition method.
FIG. 8 is another flowchart of a workflow definition method.
FIG. 9 is a structure diagram of a vital sign monitoring device.
FIG. 10 is another structure diagram of a vital sign monitoring device.

### DESCRIPTION OF THE PREFERRED EMBODIMENTS

The following provides a clear and complete description of the technical solution in the embodiments of this disclosure, in conjunction with the accompanying drawings. Obviously, the described embodiments are only some embodiments of this disclosure, not all of them. Based on the embodiments in this disclosure, all other embodiments obtained by ordinary technicians in the art without creative labor fall within the scope of protection of this disclosure.

Vital sign monitoring device is commonly used in various places, such as general wards, emergency departments, outpatient clinics, and long-term care institutions outside hospital, etc., to measure patient vital sign data or provide temporary monitoring. Although they all measure vital sign data, the workflow configuration processes of the vital sign monitoring devices may vary in different monitoring situations. The current vital sign monitoring device provides a set of main workflow configuration information. In actual use, this set of main workflow configuration information may include many redundant function items for a specific monitoring situation, such that the user should implement more operations unrelated to the specific monitoring situation, resulting in poor usability of the device. It is also possible that the function items required for this specific monitoring situation are not included, resulting in poor usability of the device.

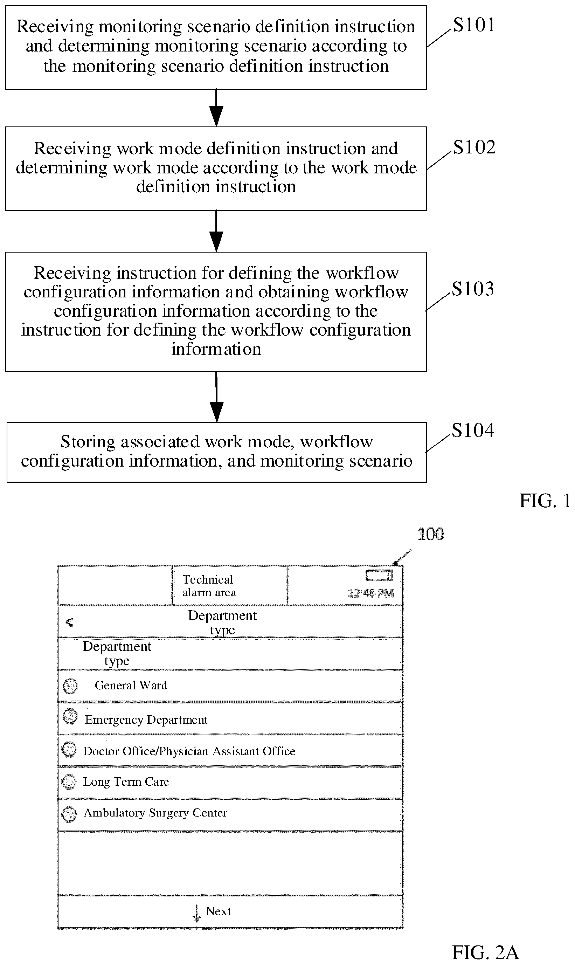

For this purpose, the embodiment of this disclosure provides a vital sign monitoring device (hereinafter referred to as monitoring device for short), which may include but not be limited to a monitor. User can customize workflow configuration information for monitoring (or workflow configuration information for short) on the vital sign monitoring device, according to monitoring requirements of the monitoring situation. FIG. 1 shows a specific process of a workflow definition method provided in an embodiment of this disclosure, including steps 101-104. The following method can be performed during a first startup setting of the vital sign monitoring device, or can be performed through a current working interface of the vital sign monitoring device. In some embodiments, the workflow customization process can be triggered through a main menu control element of the current working interface of the vital sign monitoring device.

In step S101, a monitoring situation definition instruction is received and a monitoring situation is determined according to the monitoring situation definition instruction.

The workflow configuration information corresponds to the monitoring situation. In order to satisfy the monitoring requirements of different monitoring situations, this disclosure defines the workflow configuration information used in different monitoring situations. Therefore, before or after defining the workflow configuration information, it is necessary to determine the corresponding monitoring situation for the defined workflow configuration information. Specifically, the monitoring situations include at least one of followings: department type and business application situation.

The department type is a specific position where the vital sign monitoring device is applied. When applied in hospitals, the department type can be defined according to a classification standard of the hospital. For example, in an embodiment, the department type includes at least one of: area, department, or ward. For example, in the FDA region or the United States region, the department type includes: General Ward, Emergency Department, Doctor Office, Long Term Care, and Ambulatory Surgery Center (ASC). In CE region or Europe region, the department type includes: General Ward, Emergency Department, Physician Office, and ASC. In China region, the department type includes: General Ward, Emergency Department, Community Health Service Center, ASC, and Neonatal Department. In practical applications, different department types can be displayed according to different regions as monitoring situations for the user to select. The monitoring situation can be set as a default situation at the factory. In one embodiment, the default monitoring situation is a General Ward. The monitoring situation definition instruction is correspondingly associated with the above monitoring situation.

Furthermore, this method can further include step S 1011, in which a department type setting instruction is received to determine a department type which is used by the device.

The business application situation is an actual business situation in which the vital sign monitoring device is applied, and is generally determined according to an actual business requirement of a monitored object. For example, the business application situation can include at least one of followings: an initial hospital admission assessment situation, a general round situation, a triage situation of Emergency Department, etc., a continuous monitoring situation in General Ward, a temporary continuous monitoring situation, etc.

This method can further include step S1012, in which a business application situation definition instruction is received to determine a business application situation.

It can be understood that in some embodiments, the monitoring situation can only include the department type, and the department type can correspond to the workflow configuration information separately. In some embodiments, the monitoring situation can only include the business application situation, and the business application situation can correspond to the workflow configuration information separately. In some embodiments, due to the possible correlation between the business application situation and the department type, the business application situation can be further assigned to the department type. Multiple business application situations can be set under one department type, and each business application situation has corresponding workflow configuration information. For example, in a General Ward, the vital sign monitoring device may have different business application situations, such as initial hospital admission assessment, daily general round, and temporary continuous monitoring. The initial hospital admission assessment situation corresponds to first preset workflow configuration information, daily general round corresponds to second preset workflow configuration information, and temporary continuous monitoring corresponds to third preset workflow configuration information. The user can establish initial workflow configuration information by selecting the business application situation used by the current device. Alternatively, based on the default preset workflow configuration information, the user can edit and modify one or more of the preset workflow configuration information as required. Optionally, the user can also add or delete the preset workflow configuration information as required.

The monitoring situation can also include situation names customized by the user based on the actual situation, which is not specific defined herein.

In an embodiment, the vital sign monitoring device includes a human-machine interaction interface, which is configured to display information, which at least includes a monitoring situation definition area, wherein the monitoring situation definition area 100 includes at least one of a department type definition area and a business application situation definition area. The department type definition area is configured to receive a department type definition instruction, and the business application situation definition area is configured to receive a business application situation definition instruction. In some embodiments, the business situation defined by the business application situation definition instruction is bound to a predetermined workflow. When selecting this business situation, the predetermined workflow bound to it is selected as the workflow for the vital sign monitoring device.

In an embodiment, the method for determining the monitoring situation can also be achieved through other manners. For example, the processor determines environment information of the vital sign monitoring device; and then determines the monitoring situation according to the environment information. The environment information includes one or more of: voice data, text data, image data, and device position. Specifically, the user can input voice data into the monitoring device, or the monitoring device can scan text to obtain text data or scan an image to obtain image data, or the monitoring device can automatically perform regional location to obtain device position. Such that, the processor then extracts vocabulary related to a type and/or region of medical institution as a monitoring situation through technologies, such as voice recognition, text recognition, image recognition, and position recognition, etc.

In step S102, a working mode definition instruction is received and a working mode is determined according to the working mode definition instruction.

Wherein, the working mode definition instruction can be inputted into the monitoring device by the user, and the working mode definition instruction is configured to determine the working mode to which the defined workflow configuration information belongs. The working mode is defined according to continuous monitoring characteristics of the monitoring device. In some embodiments, the working mode includes a continuous measurement working mode and a discontinuous measurement working mode. In some embodiments, the discontinuous measurement working mode specifically includes a spot check mode, and the continuous measurement working mode includes a continuous monitoring mode. It can be understood that in other embodiments, the working mode can also include other modes, as long as they are distinguished according to the actual monitoring requirements of the monitoring device.

The spot check mode refers to a single measurement of vital sign data for a patient or a measurement of vital sign data for a patient at a certain moment, according to requirements. The continuous monitoring mode refers to a continuous measurement and monitoring of vital sign data for a patient over a long period of time for abnormal changes. Specifically, the continuous monitoring mode automatically acquires vital sign data of the patient at certain time intervals, such as 1 millisecond, 1 second, 5 minutes, 30 minutes, 6 hours, 12 hours, and so on. Furthermore, the continuous monitoring mode can be further divided according to a length of the time interval.

In some embodiments, different department types can be displayed according to different regions, as monitoring situations for the user to select. The working mode can be set to the default working mode at the factory. In one embodiment, the default working mode is a General Ward. In some embodiments, the default working mode can be modified, deleted, edited, or stored. In some embodiments, when switching the monitoring situation, the measurement mode set within said monitoring situation is automatically cleared.

In some embodiments, the monitoring situation includes a measurement region, which includes at least one of: the United States, Europe, and China. In some embodiments, the monitoring situation includes at least the above mentioned department types.

In some embodiments, the department type includes at least a General Ward and an Emergency Department. When the monitoring situation is General Ward, the continuous measurement working mode includes a continuous monitoring mode, while the discontinuous measurement working mode includes a general round mode and an initial evaluation mode. In some embodiments, a monitoring page of the initial evaluation mode includes an orthostatic hypotension evaluation tool.

When the monitoring situation is Emergency Department, the continuous measurement working mode includes a continuous monitoring mode, while the discontinuous measurement working mode includes a triage mode and a spot check mode.

The department type further includes one of followings: Doctor Office, Long Term Care, ASC, Community Health Service Center, and Neonatal Department.

When the department type is Doctor Office or Community Health Service Center, the continuous measurement working mode includes a continuous monitoring mode, while the discontinuous measurement working mode includes a spot check mode. In some embodiments, when the department type is Doctor Office or Community Health Service Center, a monitoring page under the spot check mode includes a height display area, a weight display area, and an average blood pressure tool.

When the department type is Long Term Care, the continuous measurement working mode includes a continuous monitoring mode, while the discontinuous measurement working mode includes a general round mode and an initial evaluation mode. Due to the similarity between the application situations of Long Term Care and General Ward, the only difference between them is inside or outside hospital. Therefore, the working mode settings of the two are also similar.

When the department type is ASC, the continuous measurement working mode includes a continuous monitoring mode, while the discontinuous measurement working mode includes a recovery mode. In some embodiments, a monitoring page of the recovery mode includes a parameter display area for blood pressure, wherein blood pressure measurement is preset with a preset time interval between two measurements, as well as with a preset measurement duration and/or preset measurement frequency for the two measurements implemented according to the preset time interval. In some embodiments, a monitoring page of the recovery mode includes a parameter display area for carbon dioxide.

When the department type is Neonatal Department, the continuous measurement working mode includes a continuous monitoring mode, while the discontinuous measurement working mode includes a spot check mode. In some embodiments, when the department type is Neonatal Department, a monitoring page includes a CCHD (Cyanotic Congenital Heart Disease) monitoring tool, a blood oxygen display area, and a pulse display area.

Different working modes can correspond to different types of monitoring situations. In one embodiment, the business application situation corresponding to the spot check mode includes: an initial hospital admission assessment situation, a spot check situation of General Ward, a triage situation of Emergency Department, etc., while the business application situation corresponding to the continuous monitoring mode includes a General Ward continuous monitoring situation, a temporary continuous monitoring situation, etc.

The continuous monitoring mode refers to continuous measurement of the vital sign parameter of the patient over a period of time. In this mode, abnormal changes in the vital signs of the patient can be monitored and an alarm notification can be given. The spot check mode refers to single measurement of the vital sign parameter of the patient, in which the single measured vital sign parameter can be stored and further transmitted to other devices. The general round mode refers to spot-checking vital sign parameters of multiple patients in sequence, and in this mode, the vital sign parameter of a single patient can be transmitted to other devices. The initial evaluation mode refers to a single measurement of vital sign parameter for the patient, for obtaining an initial assessment of physical situation according to the measurement result, such as an initial assessment for risk of falls. The triage mode refers to a single measurement of vital sign parameter for the patient to assess critical degree or level of the patient, so as to determine department to be visited by the patient. The recovery mode refers to a single measurement of vital sign parameter for the patient before or after surgery, so as to evaluate or confirm patient situation before or after surgery.

It should be noted that the specific implementation of the same working mode for different monitoring situations can be set according to requirements of each monitoring situation, and may not be absolutely the same.

The workflow can customized corresponding to the monitoring situation under different monitoring situations. In different monitoring situations, the working modes executed by the monitoring device can also vary. Therefore, the working mode of the monitoring device can be determined first, and then the monitoring situation can be determined. Optionally, the monitoring situation can be determined firstly and then the working mode of the monitoring device can be determined.

One specific way to determine the working mode is to input a working mode definition instruction to the monitoring device by the user. The working mode definition instruction is configured to indicate a working mode which the user wants to set, such that when receiving the working mode definition instruction, the monitoring device determines the working mode indicated by the instruction as its working mode. Wherein, the user can input the working mode definition instruction through various methods, such as physical button, touch mode, voice, etc. For example, in order to facilitate user to input the working mode definition instruction, the monitoring device can display a working mode definition area 200, and receive the working mode definition instruction inputted by a device operator, based on the working mode definition area 200. Specifically, the working mode definition area 200 includes options for working modes, such as a spot check mode option and a continuous monitoring mode option. When a user selects a certain option, it means that the user inputs a working mode definition instruction to the monitoring device. The monitoring device determines the working mode corresponding to the selected option as the working mode.

There is no fixed sequential relationship between the determination of the working mode and the determination of the monitoring situation, which can be arranged arbitrarily or simultaneously. Furthermore, there is no fixed sequential relationship between the determination of the working mode, the determination of the department type, and the determination of the business application situation, and the three can be arranged arbitrarily or simultaneously.

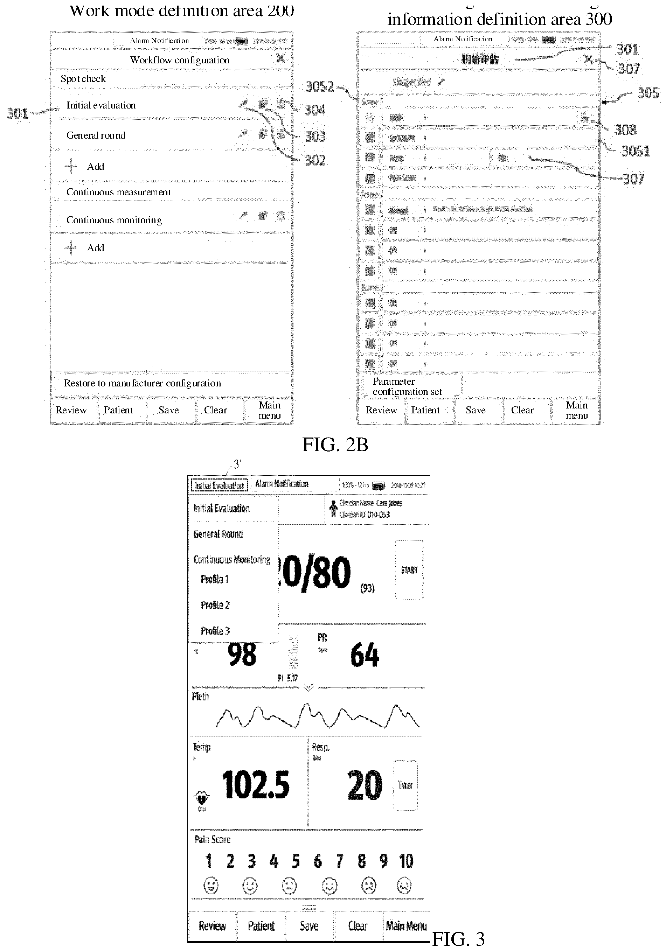

A specific determination method is as follows. When determining the working mode, an option for at least one optional monitoring situation, which is associated with the working mode, is displayed, and the device operator can select any one optional monitoring situation, and the monitoring device responds to the received selection instruction to determine the optional monitoring situation which corresponds to the selection instruction as the monitoring situation in the working mode. The monitoring situation definition area 100 and the working mode definition area 200 can at least partially overlap with each other, as shown in FIG. 2B, which illustrates an example of the monitoring situation definition interface or working mode definition interface. This interface includes two working modes: a spot check mode and a continuous monitoring mode. There are two optional monitoring situations which are associated with the spot check mode, that is, an initial evaluation situation and a general round situation. There is one optional monitoring situation which is associated with the continuous monitoring mode, that is, a continuous monitoring situation. The user can select any optional monitoring situation as the monitoring situation.

It should be noted that in the implementation shown in the diagram, the selection for monitoring situation of the user also indicates that the user has selected the working mode associated with the monitoring situation as the target working mode. It can be understood that in this embodiment, each monitoring situation is associated with its working mode, and the user selects both the working mode and the monitoring situation once, which is convenient for the user to operate and simplifies the operation steps. In one embodiment, the vital sign monitoring device displays the working mode definition area and the monitoring situation definition area. The working mode definition area and the monitoring situation definition area are the same area, and the user can complete the setting of the working mode and monitoring situation by clicking the touch screen once. Of course, the monitoring situation and working mode can also be selected separately, not limited to FIG. 2B. In addition, this determination method allows the user to select specific monitoring situation from various optional monitoring situations through the interface or the area on the interface, making the operation simple and convenient.

In some embodiments, the sequence of steps S1011, S1012, and S102 is not limited and can be arranged according to the actual situation arbitrarily, such as being executed in order, each two being executed at the same time, or even all being executed at the same time. In a specific embodiment, as shown in FIG. 2A, step S1011 is executed through the department type definition area, in which the department type setting instruction is received to determine the department type used by the device. Then, as shown in FIG. 2B, step S1012 is executed through the business application situation definition area, in which the business application situation definition instruction is received to determine the business application situation. Meanwhile, step S 102 is executed through the working mode definition area 200 which is partially overlapped with the business application situation definition area, in which the working mode definition instruction is received to determine the working mode based on the working mode definition instruction. It can be understood that at this point, the business application situation is further assigned to each corresponding working mode. The working mode definition area 200 and the business application situation definition area are the same area or are overlapped areas. Therefore, when defining the business application situation, the working mode is defined. It can be understood that the department type definition area, application situation definition area, and working mode definition area 200 can also be set as the same area or overlapped areas. When one of them is selected, the other two are automatically selected to facilitate the user setting. In some embodiments, the optional monitoring situation is bound with predefined workflow configuration information. When the optional monitoring situation is selected, the corresponding predefined workflow configuration information is selected. Alternatively, in some embodiments, the predefined workflow configuration information corresponding to the monitoring situation is named after a name of the monitoring situation, for example, "initial evaluation" and "general round" in FIG. 2B are both names of the predefined workflow configuration information and the monitoring situation. In some embodiments, the names of these predefined workflow configuration information can be set to be immutable.

Another specific determination method is that when the monitoring device determines the working mode, the user can customize specific workflow configuration information under that working mode through an adding manner. The workflow configuration information corresponds to the monitoring situation. For example, if a user inputs a name of customized workflow configuration information through an input device, such as a touch screen, the name represents a customized monitoring situation of the user, and the monitoring device determines the customized monitoring situation as the monitoring situation. In some embodiments, storing the name of the monitoring situation by the monitoring device, indicates that the monitoring device stores the monitoring situation. As shown in FIG. 2B, the user can touch control element "Add" under the spot check mode option or the continuous monitoring mode option in the working mode definition area 200 to add desired workflow configuration information inside the selected working mode, and name the desired workflow configuration information. The current monitoring situation can be directly used as the name of the workflow configuration information. In some embodiments, when the name of the added workflow configuration information is the same as the name of the existing workflow configuration information, a name conflict indication is given and the name of the added workflow configuration information should be re-edited or the newly added workflow configuration information is confirmed to overwrite the existing workflow configuration information. It can be understood that the control element "Add" can be a control element which is indicated by a text or symbol on the touch screen, such as "+" and/or "add" shown in FIG. 2B, or can be a new function triggered by a button on the device. This determination method allows the user to freely set monitoring situations according to their actual requirements, making it more flexible and enriching the diversity of monitoring situations.

In step S 103, an instruction for defining workflow configuration information is received, and workflow configuration information under the monitoring situation and the working mode is obtained according to the instruction for defining the workflow configuration information.

The workflow configuration information is determined according to the instruction for defining the workflow configuration information, and a monitoring page is generated according to the workflow configuration information. Wherein, the working mode includes a continuous measurement working mode and a discontinuous measurement working mode. When the working mode is the continuous measurement working mode, the workflow configuration includes first workflow configuration information in the continuous measurement working mode; the processor is further configured to, in the continuous measurement working mode, determine a first parameter according to the first workflow configuration information, receive and process a signal of the first parameter according to the first workflow configuration information to obtain a first parameter measurement result. When the working mode is the discontinuous measurement working mode, the workflow configuration information includes second workflow configuration information in the discontinuous measurement working mode; the processor is further configured to, in the discontinuous measurement working mode, determine a second parameter based on the second workflow configuration information, receive and process a signal of the second parameter according to the second workflow configuration information to obtain a second parameter measurement result. A display is configured to display the first parameter measurement result on a first monitoring page according to the first workflow configuration information and/or to display the second parameter measurement result on the second monitoring page according to the second workflow configuration information. A memory is configured to store the associated working mode, workflow configuration information, and monitoring situation.

Wherein, the workflow configuration information and working mode are both configured to define the workflow for performing vital sign monitoring, and the working mode and the workflow configuration information correspond to the monitoring situation.

Specifically, the workflow configuration information used varies depending on the monitoring situation. For example, the initial hospital admission assessment situation corresponds to the workflow configuration information under the initial hospital admission assessment situation. This workflow configuration information is mainly used for the initial evaluation of vital signs of newly admitted patient or patient in the emergency department, usually measuring and paying attention to the physiological parameters of patient at hospital admission. For example, the general round situation corresponds to the workflow configuration information during the general round, which mainly involves single or intermittent multiple measurements of the patient during hospitalization. For example, the triage situation in the emergency department corresponds to the workflow configuration information of the emergency triage, which mainly involves single or multiple measurements of the patient during triage of patient under emergency situation. The continuous monitoring situation can correspond to continuous monitoring workflow configuration information in General Ward, which is configured to continuously measure and monitor the vital sign of patient in the general ward for a long time. Of course, the monitoring situation can also include others, and this disclosure does not make any specific limitations.

This step is for user to customize the workflow configuration information suitable for the monitoring situation according to characteristics of the monitoring situation.

Specifically, after determining the working mode, the monitoring device can display an area for defining workflow configuration information 300. In one embodiment, the area for defining the workflow configuration information 300 can also serve as a workflow configuration information editing interface. It should be noted that the area for defining the workflow configuration information 300 can be an area or interface independent of the working mode definition area 200 and the monitoring situation definition area 100. It can be in a different interface from the latter two or at least partially overlap with the latter two. In some embodiments, the area for defining the workflow configuration information 300 may include one or more interfaces, each interface is configured to define configuration information of the same workflow configuration information in multiple aspects. The user can input the instruction for defining the workflow configuration information into this interface. The instruction for defining the workflow configuration information can be configured to indicate the desired configuration information that the user wants to set. After the monitoring device receives the instruction for defining the workflow configuration information inputted by the device operator based on the area for defining the workflow configuration information 300, the monitoring device acquires the workflow configuration information based on this instruction. As shown in FIG. 2B, if a close control element 306 or a return control element (not shown) is set in the work configuration information definition area, triggering the close control element 306 or the return control element (not shown) can disable the functionality of the area for defining the workflow configuration information 300 and save the workflow configuration information. After setting configuration values of each option of the workflow configuration information in the area for defining the workflow configuration information 300, the user only needs to click on the close control element 306 or the return control element to save the configuration values of each option of the workflow configuration information and close the area for defining the workflow configuration information 300. This reduces the operation steps and saves operation time.

The workflow configuration information is configured to define a workflow for performing vital sign monitoring. The workflow configuration information is a static representation of workflow configuration. Combined with the working mode, a dynamic workflow can be obtained by performing a process of vital sign monitoring according to relevant definitions of the workflow configuration information. It can be understood that the workflow configuration information of the monitoring device is configured to acquire vital sign parameters of a target object. The acquired physiological parameter types, physiological parameter measurement times, physiological parameter calculation methods, physiological parameter display modes, alarm settings, and other configuration information, may be different for different workflow configuration information.

In a specific implementation, the area for defining the workflow configuration information 300 can include configuration information for each setting area, and the user can select configuration information through touch modes, etc. For example, the area for defining the workflow configuration information 300 includes one or more of the following setting areas: identifier information setting area 301, parameter layout setting area 305, parameter configuration setting area, alarm setting area, area for setting a modular parameter monitoring tool, parameter measurement result processing manner setting area, and parameter monitoring auxiliary information setting area. The user defines information for each setting area separately, and this information is configured to comprehensively define the specific workflow configuration information. Correspondingly, the instruction for defining the workflow configuration information includes at least one of the following: an identifier information configuration instruction, which is configured to input identifier information of workflow configuration information; a parameter layout configuration instruction, which is configured to input parameter layout configuration information; a parameter configuration setting instruction, which is configured to set configuration information for parameter measurement; an alarm setting configuration instructions, which is configured to set alarm related information; a modular parameter monitoring tool configuration instruction, which is configured to select modular parameter monitoring tool; a processing manner setting instruction for parameter measurement result, which is configured to input a processing manner for parameter measurement result; an auxiliary information setting instruction for parameter monitoring, which is configured to set auxiliary function information for parameter monitoring.

A first setting area, namely the identifier information setting area 301, is configured to set an identifier of the workflow configuration information, such as name of the workflow configuration information. The user can use the monitoring situation as the identifier information, such as the actual usage situation, area, department, ward, etc., or customize a situation name according to the actual situation, such as continuous measurement working mode 1, continuous measurement working mode 2, etc., which is not specific defined herein.

Furthermore, in the first setting area shown in FIG. 2B, an editing control element 302, a copying control element 303, and a deleting control element 304, are provided. The editing control element 302 can trigger an instruction for editing existing workflow configuration information. The copying control element 303 can trigger an instruction for copying existing workflow configuration information. The deleting control element 304 can trigger an instruction for deleting existing workflow configuration information. In one embodiment, the interface is further provided with a recovery control element (not shown) which can trigger am instruction for restoring existing workflow configuration information to default workflow configuration information and an exit control element (not shown) which can trigger an instruction for exiting workflow configuration information setting. It can be understood that, as shown in FIG. 2B, displaying the above control elements corresponding to each identifier information can facilitate user selection. In other embodiments, the above control elements can also be centrally arranged in one area to form an operation control element setting area for workflow configuration information (not shown). After clicking the identifier information, and then selecting the desired control elements, operation of editing, copying, and deleting can also be completed.

A second setting area, namely the parameter layout setting area 305, is configured to set parameters displayed on the monitoring page and/or display layout rules on the monitoring page, such as the displayed parameters, display position of each parameter on the monitoring page, a size of display area, and a display order. The instruction for defining the workflow configuration information includes a parameter layout configuration instruction for configuring categories and/or display rules of various parameters on the monitoring page. The parameter layout setting area 305 is configured to receive the above parameter layout configuration instruction. Furthermore, the parameter layout configuration instruction includes one or more of following instructions: an instruction for determining parameter(s); an instruction for setting a monitoring sequence of parameter; an instruction for setting a display mode of parameter measurement result. The following provides a detailed explanation of the specific content of some parameters and the corresponding instructions for defining workflow configuration information on how to set configuration information of the parameters.

As shown in FIG. 2B, the parameter layout setting area 305 includes one or more parameter setting areas 3051, which are configured to receive an instruction for determining parameter(s). In some embodiments, the parameter setting area 3051 is configured to receive an instruction for selecting parameter(s) from two or more optional parameters. In some embodiments, the parameter(s) can specifically include a first parameter corresponding to a parameter which is directly measured by the monitoring device, as well as a second parameter or nursing information corresponding to a parameter which needs to be recorded and is obtained by the user through other device or manual operation. In some embodiments, the second parameter may include a customized parameter that do not exist among the optional parameters of the monitoring device. In some embodiments, the parameter setting area 3051 is further configured to display the selected parameter. As shown in FIG. 2B, the user selects the identifier information named after the monitoring situation and displays the area for defining the workflow configuration information 300, which is configured to define the workflow configuration information corresponding to the monitoring situation. The parameter setting area 3051 includes a parameter setting control element 307, which allows the user to define the parameters required in the workflow. For example, after the user touches the parameter setting control element 307, six optional parameter configuration items including NIBP (Non-invasive Blood Pressure), Manual, SPO2&PR (oxygen saturation & pulse rate), BMI (Body Mass Index), Pain Score, and Off are displayed. The user selects one or some optional parameters from the above parameter types by selecting the configuration items, and the monitoring device determines the parameters selected by the user as the parameters to be monitored for the workflow configuration information. It can be understood that the above is only an embodiment and more clinical parameters can be included, without limitation here. In some embodiments, parameters can be directly set by clicking on the parameter setting control element 307 without setting default parameters. In other embodiments, parameters can be set by default and reset by clicking on the parameter setting control element 307. Furthermore, the parameter setting control element 307 can be positioned inside the parameter display area, allowing for parameter modification when clicking on the parameter setting control element 307. This method can provide the user with indications and references, and the user only needs to modify the default settings as required, making it convenient for the user to operate.

When multiple parameter setting areas 3051 exists, the first parameter is set through a first parameter setting control element inside the first parameter setting area. When a second parameter, which is set through the second parameter setting control element inside the second parameter setting area, is the same as the first parameter, the first parameter inside the first parameter setting area is cleared. Furthermore, there are multiple interfaces, such as a first interface (SCREEN 1), a second interface (SCREEN 2), and a third interface (SCREEN 3) as shown in FIG. 2B, the first parameter setting area is positioned inside the first interface, the second parameter setting area is positioned inside the second interface, while the setting values in other parameter setting areas in the first interface are all empty. If the first parameter is set through the first parameter setting control element inside the first parameter setting area, when the second parameter, which is set through the second parameter setting control element inside the second parameter setting area, is the same as the first parameter, the first parameter in the first parameter setting area is maintained and the second parameter inside the second parameter setting area is cleared. That is to say, at least one parameter is set inside the parameter setting area of the first interface.

In some embodiments, the parameter setting control element 307 is configured to receive an instruction for determining a customized parameter as its parameter, allowing the user to customize the parameter to be measured.

The area for defining the workflow configuration information 300 also has a control element for setting a parameter display order for receiving an instruction to set a parameter display order, as shown in FIG. 2B. The sequence of parameters in the area for defining the workflow configuration information 300 can be used to indicate the display order of the parameters. In some embodiments, the user can define the sequence of parameter acquisition during a monitoring process by adjusting the order of parameter arrangement. In other embodiments, an independent parameter monitoring sequence setting control element (not shown) can also be added for setting the acquisition sequence.

In an embodiment, the parameter layout setting area includes at least one page of monitoring page layout setting area, and each page of monitoring page layout setting area includes a display setting line with a preset number of display line(s). The parameter display area is preset with an occupied width, and a number of parameters displayed in the display area setting line is determined based on a line width of the display setting line and the occupied width.

In some embodiments, the occupied width of each parameter on monitoring page is shown in the table below.

| Parameter display area | Spot check mode | continuous monitoring mode | Occupied width |
|---|---|---|---|
| NIBP | Yes | Yes | Entire line |
| SPO2+PR | Yes | Yes | Entire line, do not exist at the same time with Single PR |
| Single PR | Yes | Yes | Half or entire line, do not exist at the same time with SPO2+PR |
| SPO2b | Yes | Yes | Entire line |
| Temp | Yes | Yes | Half or entire line |
| RR | Yes | Yes | Half or entire line, do not exist at the same time with CO2+RR |
| CO2+RR | No | Yes | Entire line, do not exist at the same time with RR |
| Parameter list | No | Yes | Half or entire line |
| EWS | Yes | Yes | Entire line (license) |
| PAIN | Yes | Yes | Entire line |
| Height/Weight/BMI | Yes | No | Entire line (only doctor office) |
| Manual parameter | Yes | Yes | Entire line |

In an embodiment, each monitoring page can lay out up to 4 lines of parameters. Each line can layout a parameter that supports the entire line. Each line can layout one parameter that supports a half line. Each line can layout two parameters that support a half line. In some embodiments, one or more monitoring pages can be set without limitation. In some embodiments, at least one parameter is set on the first monitoring page. Furthermore, a control element for setting a parameter display area 3052 is provided for receiving an instruction to set a display position of a parameter measurement result. The control element for setting the parameter display area 3052 can be independently displayed on the interface, and configured to select a serial number of the monitoring page where the parameter measurement result is positioned, such as the first monitoring page (SCREEN 1), the second monitoring page (SCREEN2), and the third monitoring page (SCREEN3). In an embodiment, as shown in FIG. 2B, the control element for setting the parameter display area 3052 is a screen label, and the screen label, the parameter setting control element 307, and parameter monitoring sequence setting control element are integrated and displayed. The parameter setting area is correspondingly set under the screen label, allowing to, after selecting the parameters through the parameter setting control element 307 by the user, automatically set the monitoring order of parameters and the screen number for displaying parameter measurement results according to the parameter arrangement order from top to bottom. After the user selects the parameters through the parameter setting control element 307, the monitoring order of the parameters and the display mode of the parameter measurement results are also determined accordingly. It can be understood that all setting controls, including the control element for setting the parameter display area 3052, parameter setting control element 307, and parameter monitoring sequence setting control element, can be integrated and displayed to reduce setting operation of the user.

The parameter layout setting area 305 also includes a locking control element 308. In some embodiments, the locking control element 308 is set inside one or more parameter setting areas, as shown in FIG. 2B. The parameter configuration setting instruction also includes a locking instruction for locking a parameter display mode. In some embodiments, when the locking control element 308 is triggered, the category of the parameter is locked to the corresponding position on each monitoring page. For example, as shown in FIG. 2B, NIBP blood pressure is in an unlocked state. At this time, different parameter categories can be set in the first line of the first, second, and third interfaces. When the lock button is triggered, NIBP blood pressure is locked in the first line of the first, second, and third interfaces. After setting up, on the monitoring page, regardless of how the interface is switched, NIBP blood pressure remains being displayed at the top line position, allowing the user to pay attention to the situation of this parameter in all interfaces.

A third setting area, namely the parameter configuration setting area, is configured to set configuration information related to acquisition, processing, and display of acquired parameter signals during the execution of the workflow. For example, it can include one or more of following setting control elements: an area for setting a parameter configuration item, an area for setting a monitoring mode, an area for setting alarm configuration, and an area for setting a modular parameter monitoring tool. Correspondingly, in order to define various items of configuration information, the instruction for defining the workflow configuration information includes a parameter configuration setting instruction, which includes one or more of following instructions: an instruction for setting configuration option value of parameter, an instruction for setting monitoring mode, an instruction for setting alarm configuration information, an instruction for selecting modular parameter monitoring tool. The following provides a detailed explanation of the specific content of some parameters and how the corresponding parameter configuration instructions set the configuration information of the parameters.

The parameter configuration setting instruction includes an instruction for inputting a configuration value of a parameter, that is a configuration item definition instruction. The parameter configuration setting area includes an area for setting a parameter configuration item, which can be empty or preset with a default value. The user can directly set the configuration value during the parameter measurement process or modify the preset default value, through the configuration item definition instruction. The configuration item can include at least one of followings: parameter display color, parameter measurement interval, measurement time, measurement cycle, measurement portion, timer duration, waveform speed, waveform mode, gas concentration, sensitivity, volume, sound interval, EWS scoring system, etc. The configuration value can be text, numerical value, or a measurement of rating degree, such as, on, off, blue, green, yellow, high, medium, low, or 1, 2, 3, etc.

In some embodiments, if the user considers that the default parameter type provided cannot satisfy the requirements of daily work measurement records, the user can customize the configuration information for the user defined parameters through a control element for setting a manual parameter configuration item, according to actual requirements. By distinguishing parameter customization types, the user can further customize more parameter details. In some embodiments, parameter customization type can include numerical parameter and selective parameter. In some embodiments, the parameter configuration setting area of the numerical parameter can receive the parameter configuration setting instruction to set a unit of a parameter, a numerical input range of a parameter and a numerical input accuracy of a parameter. In some embodiments, the parameter configuration setting area of the selective parameter can receive the parameter configuration setting instruction to set parameter option content.

The parameter configuration setting instruction includes an instruction for setting a monitoring mode. The parameter configuration setting area can include an area for setting a monitoring mode, where the user selects an optional monitoring mode to define the monitoring mode of the parameter. Taking NIBP blood pressure measurement as an example, the measurement modes include routine measurement and two measurements.

Explain with NIBP blood pressure parameter. NIBP configuration option values in the continuous measurement working mode, includes: default NIBP measurement posture with available options of sitting, lying, standing, unspecified; a default NIBP measurement portion, with available options of left hand, right hand, left leg, right leg, unspecified; an NIBP measurement interval with available options of manual, 1 second, 2 seconds, 1 minute, 2 minutes, 3 minutes, 5 minutes, 10 minutes, 15 minutes, 20 minutes, 30 minutes, 1 hour, 1.5 hours, 2 hours; an NIBP measurement switch at o'clock with available options of switch-on, switch-off; an NIBP measurement sequence selection with available options of 1-5, wherein the available options for NIBP measurement sequence selection include a preset number of NIBP measurements and a time interval of each measurement within that number of measurements. NIBP configuration option values in a discontinuous measurement working mode, includes: an NIBP measurement mode with available options of once mode or twice mode. When selecting the once mode, the supported configuration item includes default NIBP measurement posture and a default NIBP measurement portion. When selecting the twice mode, the supported configuration item includes two groups of default NIBP measurement posture and default NIBP measurement portion with the options which are consistent with the once mode.

Explain with SPO2 blood oxygen parameters. Configuration option values for SPO2 include: a default sensitivity with available options of High, Med, and Low; a default waveform speed with available options of 12.5mm/s, 25mm/s; a default pulse volume with available options ranging from 0 to 10.

Explain with body temperature parameters. Configuration option values for body temperature include: a default measurement portion with available options of oral cavity, armpit, forehead, anus, unspecified.

Explain with respiratory rate RR, as a parameter which exists in a discontinuous measurement working mode. Configuration option values for respiratory rate RR include: a default timer duration with available options of 60s, 90s, 120s; a default warning tone interval with available options of 10s, 15s, 20s, 30s, 60s, and taking 15s as default.

Explain with CO2 parameter, which can only be set in a continuous measurement working mode. Configuration option values for CO2 include: a default suffocation delay with available options of 20s, 25s, 30s, 35s, 40s; a default working mode with available options of Measure, Standby; a default BTPS compensation switch with available options of switch-on, switch-off; a default waveform ruler with available options of 25, 40, 50, 60, 80 in mmHg, 10.0, 8.0, 7.0, 5.0, 3.5, 2.5, 2.0 in kPa, 10.0, 8.0, 7.0, 5.0, 3.5, 2.5, 2.0 in percentage; a default waveform speed with available options of 3mm/s, 6.25mm/s, 12.5mm/s, 25mm/s, 50mm/s; a default waveform mode with available options of line tracing, filling; a default oxygen concentration with available options ranging from 0-100, and taking 0 as default; a default laughing gas concentration with available ranging from 0-100 and taking 0 as default; a default anesthesia gas concentration, ranging from 0-24 and taking 0 as default; a default automatic standby delay with available options of Off, 15min, 30min, or 60min.

Explain with pain score parameter. Configuration option values for pain score, both in a discontinuous measurement working mode and continuous measurement working mode, include: default scoring system with available options of NEWS, MEWS, NEW2, and imported customized scoring system; scoring confirmation function switch with available options of switch-on, switch-off. The configuration option values for pain score in continuous measurement working mode, further include: an automatic scoring function switch with available options of switch-on, switch-off; an dynamic scoring refresh function switch with available options of switch-on, switch-off; an EWS alarm function switch with available options of switch-on, switch-off; etc.

The configuration option values of other parameters or other configuration option values of the above parameters can be set according to actual requirements, and there is no limit here.

The parameter configuration setting instruction includes an alarm configuration setting instruction, which is configured to set alarm configuration information. The parameter configuration setting area can include an area for setting alarm configuration, which is used for alarm configuration related information, such as alarm limits, alarm switches, and alarm level settings for each parameter. In one embodiment, the area for setting the alarm configuration only exists in continuous measurement working mode.

The parameter configuration setting instruction also includes an instruction for setting modular parameter monitoring tool. The parameter configuration setting area can include an area for setting a modular parameter monitoring tool, which is configured to provide a monitoring tool with monitoring function(s). Some monitoring situations may require special monitoring function. For example, the initial evaluation situation of a general ward requires an orthostatic hypotension evaluation tool, some European hospitals require a multiple blood pressure measurement and recording tool for initial evaluation monitoring situations, while North American clinic situations require a blood pressure average measurement tool and an irregular pulse rate tool in SPO2, etc. The user can choose the corresponding monitoring tool as the configuration information for the workflow corresponding to the monitoring situation based on the characteristics of the monitoring situation. It can be understood that the parameter monitoring tool in the area for setting the modular parameter monitoring tool can be selected by default in some monitoring situations. In an embodiment, when the monitoring situation includes a neonatal department, the CCHD monitoring tool remains in the default selected state and cannot be deleted, edited, or copied.

The fourth setting area, namely a parameter measurement result processing manner setting area, is configured to set how parameter measurement result data is processed, which can specifically include a transmission manner of the parameter measurement result. The user can define the transmission manner of the parameter measurement result by inputting the instruction for defining the workflow configuration information to the monitoring device. The monitoring result acquired by the monitoring device can be transmitted to a central monitoring station and other device(s). The transmission manner refers to through which communication manner or at when the monitoring data is transmitted to other devices. For example, according to the network environment of medical institutions, the communication manner is set as Bluetooth transmission or QR code transmission, and the transmission timing is defined as real-time network transmission or offline batch transmission.

The fifth setting area, namely a parameter monitoring auxiliary information setting area, is configured to receive a parameter monitoring auxiliary information instruction, and can include one or more of following control elements: a control element for processing manner of operator information, a control element for printing parameter. Correspondingly, the instruction for defining the workflow configuration information includes a parameter monitoring auxiliary information setting instruction, which includes one or more of followings: an instruction for setting a processing manner of operator information of a workflow, and an instruction for setting printing parameter. Specifically, workflow operators, such as nurses, may need to input their related information before operating the monitoring device. In some monitoring situations, after obtaining the parameter measurement result, the related information of the operator needs to be cleared. For example, in the initial hospital admission evaluation situation, it is often to implement the spot check for a single patient. After obtaining the physiological parameters and then storing and transmitting the same, the login information of the nurse needs to be automatically cleared. However, in other monitoring situations, it may be necessary to preserve the login information, such as in a general round situation of General Ward where multiple patients are measured one by one. After completing the physiological parameter measurement of a single patient, it is necessary to continue preserving the login information of the nurse to measure the next patient. It should be noted that in the workflows corresponding to different monitoring situations, the preserving or clearance for the information of the workflow operator can be defined by the user through the control element for processing manner of operator information.

In addition, the parameter monitoring auxiliary information setting area can also include certain special setting areas, such as an area for selecting a display that displays the parameter measurement result, and so on. It should be noted that the setting area for various workflow configuration information mentioned above is only an example and is not specifically limited in this disclosure. One skilled in this field can define configuration information related to workflow configuration information based on the actual requirements of monitoring situations. In some embodiments, the special setting area can also include a system time setting area, a parameter unit setting area, a customized network setting area, etc. In the customized network setting area, the user can set the network they use based on different protocols of the hospital.

When the working mode is a continuous measurement working mode, the workflow configuration information includes first workflow configuration information in the continuous measurement working mode, and the processor is further configured to, in the continuous measurement working mode, receive and process a signal of first parameter according to the first workflow configuration information, so as to obtain a first parameter measurement result. When the working mode is a discontinuous measurement working mode, the workflow configuration information includes second workflow configuration information in the discontinuous measurement working mode, and the processor is further configured to, in the discontinuous measurement working mode, receive and process signal of second parameter according to the second workflow configuration information, so as to obtain a second parameter measurement result. A display is configured to display the first parameter measurement result in the continuous measurement working mode, and/or the second parameter measurement result in the discontinuous measurement working mode. In some embodiments, there may be one or more displays. When there is one display, the first parameter measurement result and the second parameter measurement result can be displayed on different interfaces of the same display or in different areas of the same interface. In some embodiments, when there are more than one displays, one display is connected with the vital sign monitoring device and the other display(s) is(are) remotely connected with the vital sign monitoring device. The first parameter measurement result and/or second parameter measurement result can be synchronously displayed on all these displays. The display and human-machine interaction interface (which can be referred to as a human-machine interaction apparatus) can be the same. In some embodiments, the display is a touch screen, wherein the display and the human-machine interaction apparatus are the same device. In some embodiments, configuration values, which are in the setting areas other than the identifier information setting area 301 and in the newly added first workflow configuration information, are the same as those in the default setting areas of the first workflow configuration information in the current monitoring situation. In some embodiments, except for the identifier information setting area 301, the configuration values in the various setting areas of the newly added second workflow configuration information, are the same as those in the settings area of the default second workflow configuration information for the current department.

It should be noted that the configuration information of the workflow can be obtained through the above methods. In addition, as mentioned earlier, the monitoring device can determine the monitoring situation. In order to obtain the correlation relationship between the workflow and the monitoring situation, it is necessary to establish a corresponding relationship between the working mode, the workflow configuration information, and the monitoring situation. Due to the fact that both the workflow configuration information and working mode are configured to represent the workflow that performs vital sign monitoring, the corresponding relationship between the workflow configuration information, working mode, and monitoring situation represents the corresponding relationship between the workflow and the monitoring situation. In other words, the workflow represented by this workflow configuration information and the working mode is applicable to the workflow of the monitoring situation.

In some embodiments, after selecting the working mode by the user, the area for defining the workflow configuration information 300 can be directly displayed for the user to define the workflow configuration information. After displaying the area for defining the workflow configuration information 300, selection items for monitoring situations can be displayed for the user to determine the monitoring situation.

It can be understood that there is no order restriction between steps S101, S102, and S103, and the order in which the three definition instructions are received can be any combinations and permutations. The monitoring situation definition area 100, the working mode definition area 200, and the area for defining the workflow configuration information 300 do not have any display order restrictions. The three display areas can be displayed on the same interface or on separate interfaces, or partially overlapping with or completely independent of each other. Specifically, the three types of definition instructions can be received simultaneously or one by one, or two of which can be received simultaneously. For example, the working mode definition instruction, the monitoring situation definition instruction and the instruction for defining the workflow configuration information can be simultaneously received. Optionally, the working mode definition instruction and the monitoring situation definition instruction can be received firstly, and then the instruction for defining the workflow configuration information can be received. Optionally, the working mode definition instruction can be received firstly, then the monitoring situation definition instruction can be received, and finally the instruction for defining the workflow configuration information can be received.

In step S104, the associated working mode, the workflow configuration information, and the monitoring situation are stored.

Wherein, on the one hand, the working mode and the workflow configuration information can be stored locally on the monitoring device, and on the other hand, the working mode and the workflow configuration information can also be stored in other devices or external storage media. For example, after storing the working mode and the workflow configuration information, the working mode and the workflow configuration information can be exported through a USB flash disk or a remote control system or other devices. For example, the working mode and the workflow configuration information can be uploaded to a data management server for backup or further self-learning based on acquired multiple workflows to obtain the workflows required for other monitoring situations.

It should be noted that there is a corresponding relationship between the working mode, the workflow configuration information, and a target monitoring situation, and this corresponding relationship is stored, so that a certain monitoring situation can be positioned based on this corresponding relationship. In some embodiments, the workflow formed by the working mode and the workflow configuration information has a corresponding relationship with the target monitoring situation, and this corresponding relationship is also stored, so that a certain monitoring situation can be positioned based on this corresponding relationship.

From the above technical solution, it can be seen that the workflow definition method applied to vital sign monitoring device provided in this embodiment of this disclosure can define the working mode through inputting the working mode definition instruction to the monitoring device by the user, and input the instruction for defining the workflow configuration information to the monitoring device according to the characteristics of the monitoring situation. In this way, the monitoring device can obtain the workflow configuration according to the instruction for defining the workflow configuration information, wherein the workflow, which is presented by the workflow configuration information and the work mode, has corresponding relationship with the monitoring situation. The corresponding working mode, workflow configuration information, and monitoring situation can be stored to obtain customized workflow corresponding to the monitoring situation on the monitoring device. Through this method embodiment, the user can customize the workflow of vital sign monitoring based on the characteristics of monitoring situations under specific working modes, satisfying their requirements for specific monitoring of different monitoring situations and providing a better user experience.

It should be noted that the determination of monitoring situation is not a necessary step. In one embodiment, the implementation of this disclosure may not include step S101 (a monitoring situation definition instruction is received and a monitoring situation is determined according to the monitoring situation definition instruction).

In another embodiment provided in this disclosure, after storing the workflow, the vital sign monitoring of the patient can be implemented using the workflow. Specifically, the user or other device can transmit a trigger instruction to the monitoring device, which responds to the received trigger instruction of the workflow and executes the working mode and workflow configuration information according to the workflow.

In one implementation, the human-machine interaction apparatus can display a measurement mode identifier list, which includes multiple first measurement modes and multiple second measurement modes. The measurement mode identifier list is configured to receive an measurement mode selection instruction. The first measurement mode is determined based on a workflow configuration in the continuous measurement working mode, and the second measurement mode is determined based on a workflow configuration in the discontinuous measurement working mode. When selecting a certain measurement mode (this measurement mode is called as a target measurement mode for convenient description, wherein the target measurement mode corresponds to a target workflow configuration, that is, the target measurement mode is obtained from the target workflow configuration) from the measurement mode identifier list according to the measurement mode selection instruction, the human-machine interaction apparatus is further configured to display a target monitoring page corresponding to the target measurement mode. Wherein, the measurement mode can be called as working mode, which includes a continuous measurement working mode and a discontinuous measurement working mode.

Specifically, when the user requires to measure and record parameter(s) according to a certain measurement mode, the user inputs a measurement mode selection instruction, which is configured to select the target measurement mode from the measurement mode identifier list. One way to receive the measurement mode selection instruction can be that the vital sign monitoring device displays the measurement mode identifier list for the user. Wherein the list includes an identifier of each measurement mode, which identifier can include name of measurement situation. Selecting a target identifier in this list means that the user selects the corresponding target measurement mode, which corresponds to the target identifier. Thus, the vital sign monitoring device can determine the target measurement mode that user requires to use.

Wherein, the identifier of the measurement mode can be a name, code, or graphic symbol of the measurement mode, as long as it can refer to a unique measurement mode, and its form is not limited.

For example, FIG. 3 is a schematic diagram of a receiving manner for a measurement mode selection instruction. The user can click button 3', and the vital sign monitoring device displays a measurement mode identifier list for the user. This list includes multiple measurement mode identifiers such as "Initial Evaluation", "General Round", and "Continuous Monitoring", etc. Assuming that the target identifier selected by the user is "Initial Evaluation", the vital sign monitoring device can determine that the target measurement mode, that the user requires to use, is the measurement mode corresponding to "Initial Evaluation".

In addition, another way to receive the measurement mode selection instruction can be that the vital sign monitoring device receives the measurement mode selection instruction inputted by the user through swiping gesture in the currently displayed parameter layout page. This measurement mode selection instruction represents that the user selects a target parameter layout page from multiple parameter layout pages. For example, if a user swipes the currently displayed parameter layout page through swiping gesture, the current displayed parameter layout page is switched to the next parameter layout page, wherein the swiping gesture can be in various directions such as up and down or left and right. Due to that each parameter layout page corresponds to one measurement mode, each swiping gesture of the user is perceived by the vital sign monitoring device as a measurement mode selection instruction inputted by the user, and this instruction represents the target measurement mode which is selected by the user. When using the swiping gesture to switch the parameter layout pages and the parameter report pages, the direction of swiping gesture can be defined to restrict different switching objects. In some embodiments, the swiping gesture in a first direction is configured to switch the parameter layout pages, while the swiping gesture in a second direction is configured to switch the work interface. In some embodiments, the first direction is defined as a length direction of the vital sign monitoring device, and the second direction is defined as a width direction of the vital sign monitoring device.

In this embodiment, there are various ways to receive the measurement mode selection instruction inputted by the user, and there is no specific limitation for this herein. For example, this method can also be that the vital sign monitoring device provides an input box for the measurement mode identifier to the user, and the user enters the measurement mode identifier into this input box, so that the vital sign monitoring device determines the target measurement mode based on the input of the user.

In this embodiment, if the user considers that the currently displayed parameter layout page is not suitable for the current actual measurement situation, or considers that the currently displayed parameter layout page is not in line with the usage habits of the user, the user can switch the currently displayed measurement mode, that is, the measurement mode selection instruction includes a measurement mode switching instruction inputted by the user, which switching instruction represents switching from a current measurement mode to a target measurement mode.

In one implementation, the user determines the target workflow corresponding to the monitoring situation according to the monitoring situation and transmits a triggering instruction to the monitoring device to trigger the execution of the target workflow. In another implementation, the monitoring device can determine its current monitoring situation according to related information, and determine the target workflow corresponding to the current monitoring situation according to the corresponding relationship between the monitoring situation and the workflow, and generate a trigger instruction that trigger the execution of the target workflow. In another implementation, other devices, such as a central monitoring station, can determine the current monitoring situation of the monitoring device, and determine the target workflow corresponding to the current monitoring situation according to the corresponding relationship between the monitoring situation and the workflow, and then generate a trigger instruction that trigger the execution of the target workflow and transmit it to the monitoring device. It can be seen that monitoring device can receive the trigger instruction from the user, itself, or other devices. Furthermore, the monitoring device determines the target workflow to be executed according to the trigger instruction, and then executes the workflow based on the configuration information of the workflow.

In addition, the embodiment of this disclosure also provides a workflow definition method, including: responding to a working mode definition instruction, so as to determine a working mode, wherein the working mode includes a continuous measurement working mode and a discontinuous measurement working mode, wherein the continuous measurement working mode includes multiple first measurement modes, and the discontinuous measurement working mode includes multiple second measurement modes; responding to an instruction for defining workflow configuration information, so as to determine workflow configuration information in the working mode, and so as to determine a monitoring page according to the workflow configuration information; responding to a measurement mode selection instruction, so as to determine a target measurement mode among preset multiple first measurement modes and multiple second measurement modes; and displaying a target monitoring page which corresponds to the target measurement mode.

It should be noted that, unlike the above embodiments, the measurement mode selection instruction is not limited to be inputted through the measurement mode identifier list, but can also be inputted through other methods. In a specific implementation, a code scanner can scan identifier codes, such as QR codes and barcodes, to obtain identifier codes. QR codes and barcodes can be associated with patients or medical workers. The code scanner transmits the identifier codes to the processor of the vital sign monitoring device, and the processor determines the first measurement mode and/or second measurement mode corresponding to the identifier code as the target measurement mode. In another specific implementation, biometric recognition devices can obtain biometric information, such as fingerprints, voiceprints, etc., from users who can be patients or medical workers. The biometric recognition device transmits biometric information to the processor of the vital sign monitoring device, and the processor determines the first measurement mode and/or second measurement mode corresponding to the biometric information as the target measurement mode. In another specific implementation, other devices, such as monitoring stations, central stations, etc., transmit the measurement mode selection instruction to the processor of vital sign monitoring device, and the processor determines the first measurement mode and/or second measurement mode corresponding to the measurement mode selection instruction as the target measurement mode.

It should be noted that other explanations of this embodiment can be found in other embodiments and are not elaborated here.

The workflow definition method introduced above customizes the configuration information of the workflow by the user. In addition to this method, this disclosure also provides an embodiment of a workflow definition method for a monitoring device to automatically obtain configuration information from another device, as shown below.

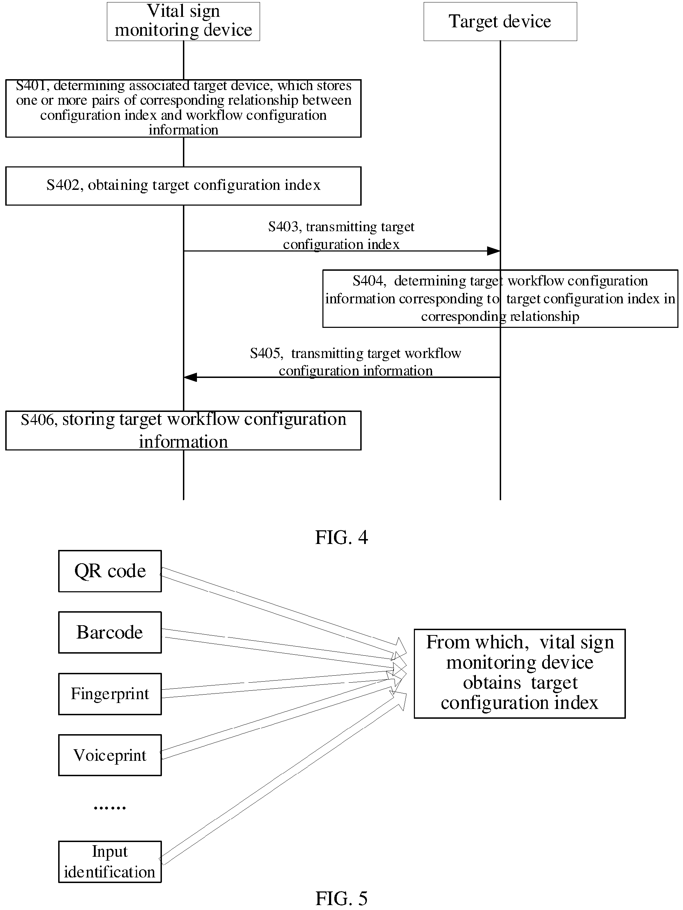

As shown in FIG. 4, the embodiment of this disclosure provides a workflow definition method, which specifically includes steps 401-406.

In step S401, vital sign monitoring device determines an associated target device, which stores one or more pairs of corresponding relationship between a configuration index and workflow configuration information.

Wherein, the monitoring device determines the target device, which requires to store the corresponding relationship between the configuration index and workflow configuration information. The configuration index, also known as a configuration identifier, is configured to uniquely represent workflow configuration information. The configuration index can be geographical position, configuration number, user ID, and so on. The configuration information on the target device can be customized by the user or obtained by self-learning through the device, and there are no specific limitations in this disclosure.

The target device can be a monitoring device or other types of devices with a defined workflow. In addition, the target device and the monitoring device executing the method can be positioned in the same geographical area or different geographical areas. If they are positioned in different geographical areas, the monitoring device can determine the device deployed in different areas as the target device. For example, monitoring devices deployed in the United States can obtain workflow configuration information from target devices which are deployed in China, so that the monitoring devices in the United States can determines the devices in the China region as target devices.

Alternatively, the workflow configuration information can be stored in external memories, such as mobile hard disks, USB disks, etc. When the monitoring device needs to define the workflow, the external memory can be communicatively connected with the monitoring device, so that the monitoring device can determine the external memory connected to its external storage interface as the target device.

In step S402, the vital sign monitoring device acquires the target configuration index.

Wherein, the target configuration index refers to an index of a target workflow that the monitoring device wants to configure. As shown in FIG. 5, the target configuration index can be obtained through various methods.

In one implementation, the configuration index can be recorded in identifier code data, such as QR codes and barcodes, which are pre-generated. The monitoring device can scan the identifier code and analyze it to extract the target configuration index from the identifier code. A specific application situation is that the target device can acquire target workflow configuration information and then generate an index of the configuration information, and further generate identifier code data from the index. If the target workflow requires to be defined on the monitoring device, the monitoring device can scan the identifier code to obtain the target configuration index, which can be used to obtain the target workflow configuration information from the target device.

In another implementation, the configuration index can specifically be identity identifier of the user, such as biometric information of fingerprints, iris, voiceprints, etc. The monitoring device can acquire biometric information of device operator and determine this biometric information as the target configuration index. A specific application situation is that the users can customize their commonly used workflow in target monitoring situations on a certain monitoring device, and input their own biometric information as the configuration index for the workflow. This monitoring device can serve as a target device. If users want to define the same target workflow on other monitoring devices, they can input their own biometric information as the target configuration index to the other monitoring devices, and the other monitoring devices automatically acquire the corresponding target workflow from the target device according to the target configuration index.

In another implementation, the user can directly input the identifier of the desired target workflow configuration information to the monitoring device. The monitoring device receives the identifier of the configuration information and uses it as the target configuration index. Specifically, the monitoring device can provide identifiers for the workflow configuration information stored by the target device, allowing users to select a certain identifier for inputting the desired target workflow configuration information.

In another implementation, the configuration index of workflow configuration information is information that can represent a type and/or region of a medical institution. The workflow represented by this type of workflow configuration information is applicable to monitoring situations related to medical institutions and/or geographical positions. For example, if emergency rooms in North America require a specially customized workflow, the workflow information applicable to emergency rooms in North America takes the region and medical institution type as the configuration index. Therefore, if the medical device wants to define this type of workflow, it can extract information related to the type and/or region of the medical institution from the received information when determining the target configuration index, and uses the extracted information as the target configuration index. For example, the user can input voice data into the monitoring device, or the monitoring device can scan texts or images, or the monitoring device can automatically locate its region, and then extract vocabulary related to the type and/or region of the medical institution as the target configuration index, through technologies such as voice recognition, text or image recognition, and position recognition.

The above methods are illustrative examples, and the monitoring device can also acquire the target configuration index through other methods. Furthermore, it should be noted that the execution order of steps 401 and 402 is not limited to the one shown in FIG. 4, and can also be that the step S402 is followed by the step S401 or both steps S401 and S402 are implemented at the same time.

In step S403, the vital sign monitoring device transmits the target configuration index to the target device.

Wherein, the monitoring device is communicatively connected with the target device, and after determining the target configuration index, the monitoring device can transmit the target configuration index to the target device through this communicative connection.

In step S404, the target device determines the target workflow configuration information corresponding to the target configuration index in the corresponding relationship.

Wherein, the target device stores one or more pieces of workflow configuration information, and each piece of workflow configuration information has a corresponding configuration index. The target configuration index is configured to indicate at least one of the target working mode and target workflow configuration information, which corresponds to the target configuration index in the corresponding relationship and is determined by the target device. For the convenience of description, the corresponding workflow configuration information can be referred to as target workflow configuration information.

In step S405, the target device transmits the target workflow configuration information to the vital sign monitoring device.

In step S406, the vital sign monitoring device stores the target workflow configuration information.

Wherein, the monitoring device receives the target workflow configuration information and stores it locally. It should be noted that the workflow configuration information on the target device corresponds to the monitoring situation, and the target workflow configuration information stored by the monitoring device also corresponds to a certain monitoring situation, and this monitoring situation is the target monitoring situation in the above embodiment. It should be noted that in one embodiment, the workflow configuration information does not correspond to the monitoring situation. In another embodiment, after receiving the target workflow configuration information, the monitoring device may not save it, but display the monitoring interface corresponding to the target workflow configuration information, which can be called as the target monitoring interface.

From the above technical solution, it can be seen that some workflow configuration information can be stored in advance on the target device. The monitoring device acquires the desired workflow from the target device through the configuration index, thereby achieving automatic definition of workflow on the monitoring device and simplifying user configuration operation. And through the target device, batch configuration of the workflow can be performed on multiple monitoring devices, resulting in higher workflow configuration efficiency.

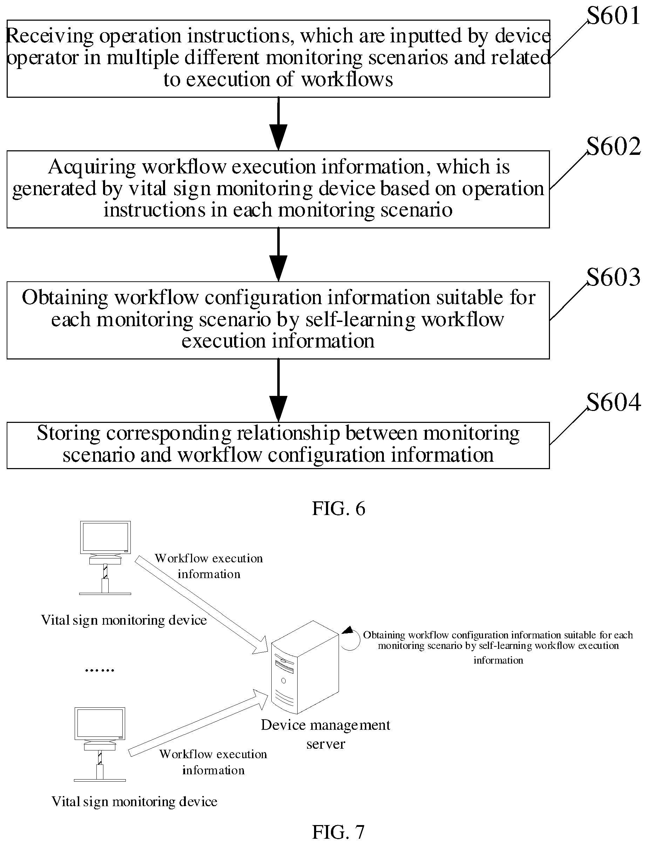

In order to further simplify user definition operation, the embodiment of this disclosure also provides a workflow definition method applied to a monitoring device, for customizing the workflow through self-learning. As shown in FIG. 6, the workflow definition method can specifically include steps 601-604.

In step S601, operation instructions, which are inputted by a device operator in multiple different monitoring situations and related to execution of workflows, are received.

It can be understood that the device operator can monitor vital signs of patient by operating the monitoring device. If the monitoring situations are different, the operation instructions implemented by the device operator on the monitoring device are different. It should be noted that the monitoring device in this embodiment can obtain self-learning workflows suitable for different monitoring situations according to the operation instructions executed in various monitoring situations. Therefore, the monitoring device requires to be used in various different monitoring situations to be triggered to execute various operation instructions. The more sufficient data acquired by monitoring device, the higher the reliability of its self-learning results.

In step S602, workflow execution information, which is generated by a vital sign monitoring device based on the operation instructions in each monitoring situation, is acquired.

It can be understood that the monitoring device can perform relevant actions based on the operation instructions. For example, in the initial hospital admission evaluation situation, the nurse triggers the monitoring device to measure various physiological parameters, such as blood pressure, body temperature, heart rate, blood oxygen, pulse, respiration, etc. of the patient. The monitoring device controls physiological parameter acquisition accessory to acquire various physiological parameter data of the patient through a series of instructions. The monitoring device can record the workflow information it executes.

In step S603, the workflow suitable for each monitoring situation is obtained by self-learning the workflow execution information.

Wherein, the workflow includes at least one of the following: working mode and workflow configuration information. The workflow configuration information is configured to define the workflow configuration for performing the vital sign monitoring.

Due to the fact that operation instructions are inputted by the device operator to the monitoring device, and generated by the device operator based on the characteristics of the monitoring situation, the series of operation instructions received by the monitoring device are related to a specific monitoring situation. For the monitoring device, when receiving the operation instruction, it never determines the current monitoring situation, but obtains the corresponding workflow and the monitoring situation applicable to the workflow through acquiring and analyzing a large amount of execution information triggered by operation instructions, and self-learning the corresponding working mode and/or workflow configuration information of these operation instructions. That is to say, the monitoring device obtains the monitoring situation and corresponding workflow through self-learning.

The method of self-learning can be machine learning algorithms in artificial intelligence such as neural networks, which is not elaborated here.

In step S604, corresponding relationship between the monitoring situation and workflow, is stored.

Wherein, the monitoring device can store the corresponding relationship locally, or store the corresponding relationship on external storage media or other devices.

From the technical solution, it can be seen that the workflow definition method provided in this embodiment allows the device operator to input a series of operation instructions on the monitoring device based on the characteristics of the monitoring situation. The monitoring device acquires the workflow information executed based on the operation instructions, obtains the workflow and the monitoring situation applicable to the workflow through self-learning the workflow information, thus achieving automatic configuration of the workflow.

Alternatively, the information acquired by the monitoring device not only includes workflow execution information, but also related information about the monitoring situation corresponding to the workflow execution information. In other words, the monitoring device does not require to obtain the monitoring situation corresponding to the workflow execution information by self-learning, but only requires to obtain the workflows under each monitoring situation according to the workflow execution information, by self-learning.

In addition, the embodiment of this disclosure also provides a workflow definition method, including:
receiving an operation instruction, which is inputted by a device operator in a predetermined working mode and related to execution of a workflow; wherein the predetermined working mode includes a continuous measurement working mode or a discontinuous measurement working mode; and acquiring workflow execution information, which is generated by a vital sign monitoring device during the execution of the workflow in the predetermined working mode based on the operation instruction; obtaining recommended workflow configuration information, which corresponds to the predetermined working mode, by self-learning the workflow execution information; and displaying a recommended monitoring page which corresponds to the recommended workflow configuration information.

Specifically, after self-learning the workflow execution information, the workflow configuration information corresponding to the predetermined working mode can be obtained and intuitively displayed to the user for confirmation or modification. Therefore, this workflow configuration information can be referred to as recommended workflow configuration information. In order to facilitate intuitive confirmation or modification by the user, the recommended workflow configuration information is displayed on a page that can be referred to as the recommended monitoring page.

It should be noted that other explanations related to this embodiment can be found in the related explanations of the previous embodiment, and is not repeated here. In this embodiment, the workflow execution information may not correspond to the monitoring situation. Accordingly, the generated recommended workflow configuration information may not correspond to the monitoring situation, and the recommended monitoring page may not correspond to the monitoring situation.

Furthermore, the monitoring device can determine its currently executed workflow configuration information and/or working mode based on the self-learning results, and output indication information for adjustment when determining different. Specifically, the current monitoring situation, as well as currently executed workflow configuration information and/or working mode of the vital sign monitoring device, are monitored; the target workflow configuration information and/or target working mode corresponding to the current monitoring situation are/is determined according to the corresponding relationship; if the currently executed workflow configuration information is different from the target workflow configuration information defined by the target workflow configuration information, indication information is displayed, which can include desired target workflow configuration information and switching control element, in response to a workflow configuration information switching instruction inputted by the device operator according to the indication information, the currently executed workflow configuration information is switched to the desired target workflow configuration information; and/or, if the currently executed working mode is different from the target working mode, an indication information is displayed, which can include a desired target working mode and switching control element, in response to an working mode switching instruction inputted by the device operator according to the indication information, the currently executed working mode is switched to the desired target working mode. It should be noted that the determination method of the current monitoring situation can refer to the description in the above embodiments, and it is not repeated here.

In another embodiment, if the monitoring device determines that the currently executed workflow is different from the target workflow, it can switch the currently executed workflow to the target workflow to achieve automatic adjustment. This operation method is simpler and more convenient. Specifically, if the monitoring device determines that the currently executed workflow configuration information is different from the target workflow configuration information, it can switch the currently executed workflow configuration information to the target workflow configuration information. And/or, if the monitoring device determines that the current working mode is different from the target working mode, it can switch the current working mode to the target working mode. This technical solution achieves automatic adjustment and makes the operation simpler and more convenient.

The self-learning workflow configuration method provided in the above embodiments is applied to the monitoring devices. Due to the self-learning method requiring the workflow execution information acquisition in multiple monitoring situations, the application situations of a single monitoring device may not be very broad. In order to acquire as much workflow execution data as possible for self-learning, this embodiment of this disclosure provides a workflow definition method applied to a device management server. It should be noted that compared to the above embodiments, the workflow method provided in this embodiment has the same main process, but different in execution subjects.

As shown in FIG. 7, a device management server is communicatively connected with at least one vital sign monitoring device.

The monitoring device is a device with vital sign monitoring function, which can trigger a workflow for executing vital sign monitoring based on a user instruction, and generate execution information during the workflow execution process. The monitoring device transmits the workflow execution information to the device management server.

The device management server acquires the workflow execution information transmitted by various monitoring devices, obtains workflows suitable for each monitoring situation by self-learning the workflow execution information, and stores the corresponding relationship between monitoring situations and workflows.

It should be noted that in one implementation, when the monitoring device transmits the workflow execution information, it can also transmits the monitoring situation information corresponding to the workflow execution information, so that the device management server does not require to obtain the monitoring situation by self-learning. In another implementation, the monitoring device may not transmit information about the monitoring situation, such that the device management server analyzes the characteristics of the workflow execution information and determines the applicable monitoring situation for the workflow execution information.

Other explanations on self-learning workflows can be found in the above embodiments and are not elaborated here. In this embodiment, the device management server is communicatively connected with multiple monitoring devices to acquire more workflow execution information, thereby obtaining more accurate workflow configuration information by self-learning.

Furthermore, the device management server can transmit the workflow corresponding to the monitoring situation to the monitoring device according to the monitoring situation applied by the monitoring device. Specifically, the device management server determines the target monitoring situation of the target vital sign monitoring device, and determines the target workflow corresponding to the target monitoring situation according to the stored corresponding relationship; and transmits the target workflow to the target vital sign monitoring device. The vital sign monitoring devices store the target workflow information locally to obtain the workflow corresponding to the target monitoring situation.

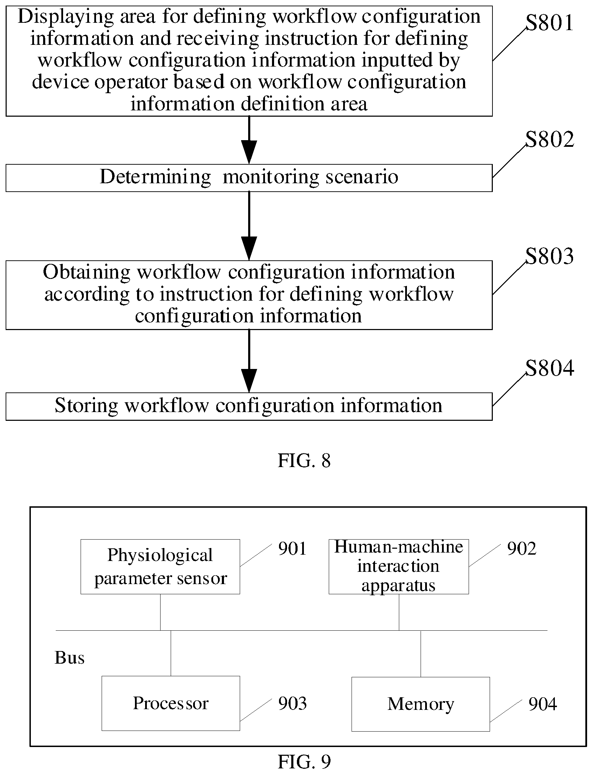

In addition, the present embodiment also provides a workflow configuration information definition method applied to a vital sign monitoring device. Compared with the first embodiment mentioned above, the present embodiment does not limit the selection of working mode when customizing workflow configuration information. As shown in FIG. 8, the specific steps of this method are as follows steps 801-804.

In step S801, an area for defining workflow configuration information is displayed and an instruction for defining workflow configuration information is inputted by a device operator based on the area for defining the workflow configuration information.

Specifically, in order to realize customization of the user for the workflow configuration information, the embodiment of this disclosure can provide an area for defining workflow configuration information, which can include various workflow configuration information items that require to be defined. The user inputs the instruction for defining the workflow configuration information through this interface, such as defining the physiological parameter type that requires to be monitored for the workflow configuration information, defining the processing manner of physiological parameter measurement result, and so on. For a specific explanation of the instruction for defining the workflow configuration information, please refer to the introduction of the above embodiments. This embodiment of this disclosure does not provide further explanation.

In step S802, a monitoring situation is determined.

Specifically, the related information of the monitoring situation can be inputted by the user, and the monitoring device can determine the monitoring situation based on the related information inputted by the user.

In step S803, workflow configuration information is obtain according to the workflow configuration information instruction.

Wherein, the workflow configuration information is configured to define the workflow configuration for performing the vital sign monitoring, and the workflow configuration information corresponds to the monitoring situation.

Specifically, the instruction for defining the workflow configuration information is inputted by the user into the monitoring device to represent the process to which the user wants to define the workflow configuration information. The monitoring device obtains the configuration information of the workflow configuration information according to the instruction for defining the workflow configuration information. For example, the physiological parameter type selected by the user in the area for defining the workflow configuration information 300 is used as the physiological parameter type to be monitored in the workflow configuration information, and the physiological parameter result display style set by the user in the area for defining the workflow configuration information 300 is set as the result display style of the workflow configuration information, etc.

It should be noted that the workflow configuration information can not only represent how the vital signs are monitored, but also how the monitoring results are displayed and processed. Any configuration information related to vital sign monitoring can be used as the workflow configuration information.

In step S804, the workflow configuration information is stored.

Specifically, the monitoring device can store the workflow configuration information locally on the device, or store the workflow configuration information on external storage media or other devices.

From the above technical solution, it can be seen that the workflow configuration information definition method provided in the embodiment of this disclosure allows the vital sign monitoring device to receive the instruction for defining the workflow configuration information from the user to determine the monitoring situation to which the workflow configuration information is applicable, establishes a corresponding relationship between the monitoring situation and the workflow configuration information, and enables the user to customize the workflow configuration information according to the monitoring situation.

In order to ensure the practical application and implementation of the above method embodiments, this disclosure provides the following embodiments of vital sign monitoring device.

As shown in FIG. 9, the embodiment of this disclosure provides a vital sign monitoring device, including:
at least one physiological parameter sensor 901, which is configured to acquire at least one vital sign parameter of a monitored object;
a human-machine interaction apparatus 902, which is configured to display information, which information at least includes a working mode definition area 200, a monitoring situation definition area 100, and an area for defining workflow configuration information 300, wherein the human-machine interaction apparatus 902 is further configured to receive a working mode definition instruction which is inputted based on the working mode definition area 200, and a monitoring situation definition instruction which is inputted based on the monitoring situation definition area 100, and an instruction for defining workflow configuration information which is inputted based on the area for defining the workflow configuration information 300; and
a processor 903, which is configured to at least determine a working mode according to the working mode definition instruction, determine the monitoring situation based on the monitoring situation definition instruction and acquire workflow configuration information according to the instruction for defining the workflow configuration information; wherein the workflow configuration information is configured to define a workflow configuration for performing vital sign parameter monitoring. It can be understood that the workflow configuration is the workflow configuration under the monitoring situation.

Wherein, the working mode includes a continuous measurement working mode and a discontinuous measurement working mode. When the working mode is the continuous measurement working mode, the workflow configuration information includes first workflow configuration information in the continuous measurement working mode, the processor is further configured to, in the continuous measurement working mode, receive and process a signal of a first parameter according to the first workflow configuration information to obtain a first parameter measurement result. When the working mode is the discontinuous measurement working mode, the workflow configuration information includes second workflow configuration information in the discontinuous measurement working mode; the processor is further configured to, in the discontinuous measurement working mode, receive and process a signal of a second parameter according to the second workflow configuration information to obtain a second parameter measurement result.

The vital sign monitoring device further includes a display, which is configured to display the first parameter measurement result and/or the second parameter measurement result, and a memory 904, which is configured to store the working mode and the workflow configuration information as a workflow, which workflow is associated with the monitoring situation.

The area for defining the workflow configuration information 300 includes one or more of following setting areas: an identifier information setting area 301, a parameter layout setting area 305, a parameter configuration setting area, a parameter measurement result processing manner setting area, and a parameter monitoring auxiliary information setting area.

In one possible implementation, the parameter configuration setting area includes one or more of following setting areas: an area for setting a parameter configuration item, an area for setting a monitoring mode, an area for setting alarm configuration, and an area for setting a modular parameter monitoring tool.

The instruction for defining the workflow configuration information includes one or more of followings: an instruction for inputting configuration option value for a parameter; an instruction for setting monitoring mode for a parameter, an instruction for setting alarm configuration information; and an instruction for selecting a modular parameter monitoring tool.

In one possible implementation, the parameter monitoring auxiliary information setting area includes one or more of following control elements: a control element for processing manner of operator information, a control element for printing parameter. Correspondingly, the instruction for defining the workflow configuration information includes one or more of followings: an instruction for setting a processing manner of operator information of a workflow, and an instruction for setting printing parameter.

In one possible implementation, the processing manner of the parameter measurement result includes a transmission manner of the parameter measurement result. Correspondingly, the instruction for defining the workflow configuration information includes an instruction for setting a transmission manner of the parameter measurement result.

In one possible implementation, the human-machine interaction apparatus is further configured to display at least one optional monitoring situation which is associated with the working mode; receive an selection instruction for an optional monitoring situation from the device operator. When determining the monitoring situation in the working mode, the processor is specifically configured to determine the optional monitoring situation corresponding to the selected instruction, as the monitoring situation in the working mode.

In one possible implementation, the processor is further configured to execute the workflow according to the workflow configuration information in response to a received trigger instruction of the workflow.

In addition, the embodiment of this disclosure also provides a vital sign monitoring device, including:
at least one physiological parameter sensor, which is configured to acquire at least one vital sign parameter of a monitored object;
a processor, which is configured to at least determine a target device which is associated with the vital sign monitoring device, wherein the target device stores one or more pairs of corresponding relationship between configuration indexes and workflow configuration information; to acquire a target configuration index; and to store a target workflow to a memory;
a communication interface, which is configured to transmit the target configuration index to the target device, wherein the target configuration index is configured to indicate the target device to determine the target workflow corresponding to the target configuration index in the corresponding relationship; wherein the communication interface is further configured to receive the target workflow returned by the target device;
the memory, which is configured to store the target workflow.

In one possible implementation, the monitoring device also includes a code scanner, which is configured to scan an identifier code generated previously. When obtaining the target configuration index, the processor is specifically configured to analyze the identifier code to extract the target configuration index from the identifier code.

In one possible implementation, the monitoring device also includes a biometric information acquisition apparatus, which is configured to acquire target biometric information of device operator. When obtaining the target configuration index, the processor is specifically configured to determine the target biometric information as the target configuration index.

In one possible implementation, the monitoring device also includes a target device which is determined by the processor as being associated with the vital sign monitoring device, and the processor is specifically configured to determine a device which is deployed in different areas of the vital sign monitoring device as the target device which is associated with the vital sign monitoring device.

In one possible implementation, the monitoring device also includes a communication interface, which includes an external memory interface. The external memory interface is configured to establish communication connection with the external memory. When determining the target device associated with the vital sign monitoring device, the processor is specifically configured to determine the external memory connected to the external storage interface as the target device.

In addition, the embodiment of this disclosure also provides a vital sign monitoring device, including:
at least one physiological parameter sensor, which is configured to acquire at least one vital sign parameter of a monitored object;
a human-machine interaction apparatus, which is configured to display information and receive operation instructions, which are inputted by a device operator in multiple different monitoring situations and related to execution of a workflow;
a processor, which is configured to acquire workflow execution information, which is generated by a vital sign monitoring device based on the operation instructions in each monitoring situation, to obtain a workflow which is suitable for each monitoring situation by self-learning the workflow execution information; wherein the workflow includes at least one of followings: a working mode and workflow configuration information; and
a memory, which is configured to store a corresponding relationship between the monitoring situation and the workflow.

In one possible implementation, the processor is further configured to monitor a current monitoring situation and a currently executed workflow of the vital sign monitoring device, and determine a target workflow corresponding to the current monitoring situation according to the corresponding relationship; to generate indication information if the currently executed workflow is different from the target workflow; to switch the currently executed workflow to the target workflow if a workflow switching instruction is received. The human-machine interaction apparatus is configured to display the indication information and receive the workflow switching instruction which is inputted by the device operator according to the indication information.

In addition, the embodiment of this disclosure also provides a vital sign monitoring device, including:
at least one physiological parameter sensor, which is configured to acquire at least one vital sign parameter of a monitored object;
a human-machine interaction apparatus, which is configured to display information, which information at least includes a working mode definition area and an area for defining workflow configuration information;
a processor, which is configured to at least determine a monitoring situation, to determine a working mode according to a working mode definition instruction, to obtain workflow configuration information according to an instruction for defining workflow configuration information; wherein the workflow configuration information is configured to define a workflow configuration for executing vital sign monitoring, wherein the working mode and the workflow configuration correspond to the monitoring situation; and
a memory, which is configured to store the working mode and the workflow configuration information.

In one possible implementation, the processor is further configured to determine position information of a vital sign monitoring device, and to determine an area for defining workflow configuration information corresponding to the position information in a preset area for defining workflow configuration information.

In addition, the embodiment of this disclosure also provides a vital sign monitoring device, including a processor and a human-machine interaction apparatus.

The processor is configured to perform at least following steps: responding to a working mode definition instruction, so as to determine a working mode, wherein the working mode includes a continuous measurement working mode and a discontinuous measurement working mode, wherein the continuous measurement working mode includes multiple first measurement modes, and the discontinuous measurement working mode includes multiple second measurement modes; responding to an instruction for defining workflow configuration information, so as to determine workflow configuration information in the working mode, and so as to determine a monitoring page according to the workflow configuration information; responding to a measurement mode selection instruction, so as to determine a target measurement mode among the multiple first measurement modes and/or the multiple second measurement modes

The human-machine interaction apparatus is configured to display a target monitoring page which corresponds to the target measurement mode.

Wherein, the processor is specifically configured to receive an identifier code which is transmitted by a code scanner, and determine the first measurement mode and/or the second measurement mode which correspond/corresponds to the identifier code as the target measurement mode; or to receive biometric information which is transmitted by a biometric recognition device, and determine the first measurement mode and/or the second measurement mode which correspond/corresponds to the identifier code as the target measurement mode; or to receive a measurement mode selection instruction which is transmitted by another device, and determine the first measurement mode and/or the second measurement mode which correspond/corresponds to the measurement mode selection instruction as the target measurement mode.

In addition, the embodiment of this disclosure also provides a vital sign monitoring device, including a human-machine interaction apparatus and a processor.

The human-machine interaction apparatus is configured to receive an operation instruction which is inputted by a device operator in a predetermined working mode and related to execution of a workflow; wherein the predetermined working mode includes a continuous measurement working mode or a discontinuous measurement working mode; and
a processor, which is configured to acquire workflow execution information which is generated by the vital sign monitoring device during the execution of the workflow in the predetermined working mode based on the operation instruction; to obtain recommended workflow configuration information which corresponds to the predetermined working mode, by self-learning the workflow execution information;
wherein the human-machine interaction apparatus is further configured to display a recommended monitoring page which corresponds to the recommended workflow configuration information.

In addition, the embodiment of this disclosure further provides a device management server, which is communicatively connected to at least one vital sign monitoring device, including:
a processor, which is configured to acquire workflow execution information of at least one vital sign monitoring device in multiple different monitoring situations; to obtain workflow(s) suitable for each monitoring situation, by self-learning the workflow execution information, wherein the workflow(s) includes/include a working mode and workflow configuration information;
a memory, which is configured to store a corresponding relationship between the monitoring situation and the workflow.

In one possible implementation, the processor is further configured to determine a target monitoring situation of a target vital sign monitoring device, and determine a target workflow which corresponds to the target monitoring situation. The device management server further includes a communication interface for transmitting the target workflow to the target vital sign monitoring device, wherein the target workflow includes a working mode and workflow configuration information corresponding to the target monitoring situation.

In a specific embodiment, the vital sign monitoring device may specifically include a monitor or module component, which further includes a single-parameter monitor, a multi-parameter monitor, a vital sign monitor, a general round monitor, and other multiple monitors or module components. The monitor or module component has at least one workflow in which physiological parameters of the monitored object are measured. It can be understood that in a workflow, there can be only one measurement function or multiple measurement functions. When there are multiple measurement functions, there can be sequential requirements between them. Vital sign monitoring devices can perform one or more different measurement functions in different workflows to adapt to the different measurement functions and methods required by users in different regions, situations, or departments. For example, in a Doctor Office, it is often necessary to measure average blood pressure and body health index, while in a general ward, it is not necessary to measure body health index. In a neonatal department, complex congenital heart disease function is required, and in an adult general ward, it is usually not necessary. The user can customize the configuration information under different working modes to configure different workflows according to their own requires to adapt to different measurement requirements. It can be understood that the working mode under each workflow can be the default working mode, or the configuration information under each workflow can also be the default configuration information. The user can select the default configuration information to configure the measurement functions under different workflows, or customize and save the measurement functions under different workflows. There is no restriction for this. By setting different configuration information, the user can define physiological parameters (such as body temperature, blood pressure, etc.) obtained through parameter measurement accessories at required time intervals or frequencies. It is also possible to define measurement functions that are manually entered by the user without using parameter measurement accessories, such as urine volume.

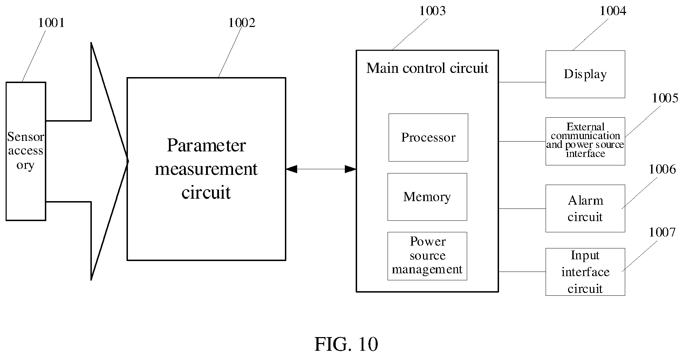

As shown in FIG. 10, a system framework diagram of a monitor or module component is provided. The monitor or module component at least includes a parameter measurement circuit 1002. The parameter measurement circuit 1002 includes a parameter measurement circuit 1002 corresponding to at least one physiological parameter, and the parameter measurement circuit 1002 includes at least one of followings parameter measurement circuits: an electrocardiogram signal parameter measurement circuit, a respiratory parameter measurement circuit, a body temperature parameter measurement circuit, a blood oxygen parameter measurement circuit, a non-invasive blood pressure parameter measurement circuit, an invasive blood pressure parameter measurement circuit, etc. Each parameter measurement circuit 1002 is connected with an externally inserted sensor accessory 1001 through a corresponding sensor interface. The sensor accessory 1001 includes detection accessories or modules corresponding to physiological parameter detection such as electrocardiogram, respiration, blood oxygen, blood pressure, body temperature detection, etc. The parameter measurement circuit 1002 is mainly used to connect the sensor accessory 1001 to obtain acquired physiological parameter signals, and can include measurement circuits for at least two or more physiological parameters. The parameter measurement circuit 1002 can be, but is not limited to, physiological parameter measurement circuit 1002 (module), physiological parameter measurement circuit 1002 (module), or sensor accessory 1001 which acquires human physiological parameters. Specifically, the parameter measurement circuit 1002 obtains physiological sampling signals related to patients acquired by the external physiological parameter sensor accessories through an extension interface, and processes them to obtain physiological data for alarm and display. The extension interface can also be used to output control signals, on how to acquire physiological parameters from the main control circuit 1003, to external physiological parameter monitoring accessories through corresponding interfaces, achieving monitoring and control of patient physiological parameters.

The monitor or module component can also include a main control circuit 1003, which needs to include at least one processor and at least one memory. Of course, the main control circuit 1003 can also include at least one of: a power source management module, power source IP module, and interface conversion circuit. The power management module is configured to control the power on/off of the entire machine, time sequence of power on/off for various power source domains on the board, and battery charging and discharging, etc. The power source IP module refers to associating the schematic diagram and PCB layout diagram of frequently revoked power circuit units into a separate power module, that is, converting an input voltage into an output voltage through a predetermined circuit, where the input voltage and output voltage are different, such as converting a voltage of 15V to 1.8V, 3.3V, or 3.8V, etc. It can be understood that the power source IP module can be single-channel or multi-channel. When the power source IP module is single channel, the power source IP module can convert one input voltage into one output voltage. When the power source IP module is multi-channel, the power source IP module can convert one input voltage into multiple output voltages, and the voltage values of multiple output voltages can be the same or different, thus satisfying the different voltage requirements of multiple electronic components at the same time. Moreover, the module has fewer external interfaces and works as a black box in the system, and is decoupled from external hardware systems, improving the reliability of the entire power system. The interface conversion circuit is configured to convert the signals outputted by the smallest system module of the main control (i.e., at least one processor and at least one memory in the main control circuit 1003) into input standard signals required by actual external devices, such as converting RGB digital signals outputted by the main control CPU into VGA analog signals for supporting external VGA display function, or converting RMII signals into standard network differential signals for supporting external network function.

In addition, the monitor or module component can also include one or more of local display 1004, alarm circuit 1006, input interface circuit 1007, external communication and power source interface 1005. The main control circuit 1003 is configured to coordinate and control various boards, circuits, and devices in the monitor or module component. In this embodiment, the main control circuit 1003 is configured to control data exchange between the parameter measurement circuit 1002 and the communication interface circuit, as well as transmission of control signals, and to transmit physiological data to the display 1004 for display. It can also receive user control instructions from physical input interface circuits, such as touch screens or keyboards and buttons, and can also output control signals on how to acquire physiological parameters. The alarm circuit 1006 can be an audible and visual alarm circuit. The main control circuit 1003 completes the calculation of physiological parameters, and transmits the calculation results and waveforms of parameters to the host (such as the host, PC, central station, etc. with the display 1004) through the external communication and power source interface 1005. The external communication and power source interface 1005 can be one or a combination of local area network interfaces composed of Ethernet, Token Ring, Token Bus, and optical fiber distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB. The external communication and power source interface 1005 may also be one or a combination of a wireless data transmission interface and a wired data transmission interface. The host may be any computer device such as a host of a monitor, an electrocardiograph, an ultrasonic diagnosis instrument, a computer, etc., which is installed with compatible software to form a monitoring device. The host can also be a communication device, such as a mobile phone, monitor, or module component, which transmits data to a mobile phone that supports Bluetooth communication through a Bluetooth interface, achieving remote data transmission.

The monitoring module component can be arranged outside the monitor housing as an independent plug-in parameter module, or can form a plug-in monitor by inserting into the host of the monitor (including the main control board), can serve as an external accessory of the monitor, or be connected with the host of the monitor (including the main control board) via a cable and taking the build-out parameter module as an external accessory of the monitor. Of course, the monitoring module component can also be built into the housing, integrated with the main control module, or physically separated and arranged within the housing to form an integrated monitor.

The interfaces, regions, and control elements in this disclosure are only illustrative and do not necessarily require or imply any inclusion relationship or order between them. In some embodiments, the interfaces, regions, and control elements can be replaced with each other. The display positions of interfaces, regions, and control elements can overlap and blend with each other, as long as they have the functions they point to.

It can be understood that, unless otherwise specified, step numbers in the embodiments are only for the convenience of pointing and explaining the content of each step, and do not limit the sequence of steps.

It should be noted that each embodiment in this specification is described in a progressive manner, and each embodiment focuses on the differences from other embodiments. The same and similar parts between each embodiment can be referred to each other.

It should also be noted that in this disclosure, relational terms, such as first and second, are only used to distinguish one entity or operation from another entity or operation, and do not necessarily require or imply any actual relationship or order between these entities or operations. Moreover, the terms "including", "comprising", or any other variation thereof are intended to encompass non-exclusive inclusion, such that a process, method, item, or device that includes a series of elements not only includes those elements, but also other elements that are not explicitly listed, or also include elements inherent in such a process, method, item, or device. Without further limitations, the elements limited by the statement "including a/the..." do not exclude the existence of other identical elements in the process, method, item, or device that includes the aforementioned elements.

The above explanation of the disclosed embodiments enables one skilled in the art to implement or use this disclosure. The various modifications to these embodiments are apparent to one skilled in the art, and the general principles defined in this disclosure can be implemented in other embodiments without departing from the spirit or scope of this disclosure. Therefore, this disclosure is not limited to the embodiments shown, but rather to the widest range consistent with the principles and novel features disclosed herein.

## Claims

1. A vital sign monitoring device, **characterized in that**, comprising:
a human-machine interaction apparatus, which is configured to display a measurement mode identifier list, a working mode definition area, and an area for defining workflow configuration information; wherein, the measurement mode identifier list comprises multiple first measurement modes and multiple second measurement modes, the measurement mode identifier list is configured to receive an measurement mode selection instruction, the working mode definition area is configured to receive a working mode definition instruction, the area for defining the workflow configuration information is configured to receive an instruction for defining the workflow configuration information; and
a processor, which is configured to:
determine a working mode according to the working mode definition instruction, wherein the working mode comprises a continuous measurement working mode and a discontinuous measurement working mode, wherein the continuous measurement working mode comprises the multiple first measurement modes, and the discontinuous measurement working mode comprises the multiple second measurement modes;
determine workflow configuration information in the working mode according to the instruction for defining the workflow configuration information, and determine a monitoring page according to the workflow configuration information; and
select a target measurement mode, which corresponds to target workflow configuration information, from the measurement mode identifier list, according to the measurement mode selection instruction;
wherein the human-machine interaction apparatus is further configured to display a target monitoring page which corresponds to the target measurement mode.

2. The vital sign monitoring device according to claim 1, **characterized in that**:
the processor is further configured to determine environment information of the vital sign monitoring device, and to determine a monitoring situation according to the environment information; wherein the environment information comprises one or more of: voice data, text data, image data, and device position;
wherein in order to determine workflow configuration information in the working mode according to the instruction for defining the workflow configuration information, the processor is specifically configured to:
determine the workflow configuration information in the working mode and the monitoring situation, according to the instruction for defining the workflow configuration information.

3. The vital sign monitoring device according to claim 1, **characterized in that**:
the human-machine interaction apparatus is further configured to display a monitoring situation definition area, which is configured to receive a monitoring situation definition instruction; and
the processor is further configured to determine a monitoring situation according to the monitoring situation definition instruction;
wherein in order to determine workflow configuration information in the working mode according to the instruction for defining the workflow configuration information, the processor is specifically configured to:
determine the workflow configuration information in the working mode and the monitoring situation, according to the instruction for defining the workflow configuration information.

4. The vital sign monitoring device according to claim 3, **characterized in that**, the monitoring situation definition area comprises a department type definition area, which is configured to define a department type; the monitoring situation comprises the department type; the department type comprises at least one of: General Ward, Emergency Department, Doctor Office, Long Term Care, Ambulatory Surgery Center, Community Health Service Center, and Neonatal Department.

5. The vital sign monitoring device according to claim 4, **characterized in that**, the department type is classified according to national region as follows:
when the national region is the United States, the department type comprises: General Ward, Emergency Department, Doctor Office, Long Term Care, and Ambulatory Surgery Center;
when the national region is Europe, the department type comprises: General Ward, Emergency Department, Doctor Office, and Ambulatory Surgery Center; and
when the national region is China, the department type comprises: General Ward, Emergency Department, Community Health Service Center, Ambulatory Surgery Center, and Neonatal Department.

6. The vital sign monitoring device according to claim 2 or 3, **characterized in that**, the working mode definition area has at least one of following scenarios:
when the monitoring situation is General Ward, the continuous measurement working mode comprises a continuous monitoring mode, while the discontinuous measurement working mode comprises a general round mode and an initial evaluation mode;
when the monitoring situation is Emergency Department, the continuous measurement working mode comprises a continuous monitoring mode, while the discontinuous measurement working mode comprises a triage mode and a spot check mode;
when the monitoring situation is Doctor Office or Community Health Service Center, the continuous measurement working mode comprises a continuous monitoring mode, while the discontinuous measurement working mode comprises a spot check mode;
when the monitoring situation is Long Term Care, the continuous measurement working mode comprises a continuous monitoring mode, while the discontinuous measurement working mode comprises a general round mode and an initial evaluation mode;
when the monitoring situation is Ambulatory Surgery Center, the continuous measurement working mode comprises a continuous monitoring mode, while the discontinuous measurement working mode comprises a recovery mode; and
when the monitoring situation is Neonatal Department, the continuous measurement working mode comprises a continuous monitoring mode, while the discontinuous measurement working mode comprises a spot check mode.

7. The vital sign monitoring device according to claim 1, **characterized in that**, the area for defining the workflow configuration information comprises one or more of following setting areas:
a workflow name setting area, which is configured to receive a workflow name setting instruction;
a parameter layout setting area, which is configured to set a parameter type and/or a parameter display layout rule, on the monitoring page; and
a parameter configuration setting area, which is configured to set at least one of followings on the monitoring page: a parameter signal acquisition way, a parameter signal processing way and a parameter measurement result display way.

8. The vital sign monitoring device according to claim 7, **characterized in that**, the monitoring page comprises at least two monitoring pages; the parameter layout setting area comprises at least two monitoring page layout setting areas, which are configured to correspondingly define the at least two monitoring pages; wherein each monitoring page layout setting area is configured to set the parameter type and/or the parameter display layout rule on one corresponding monitoring page;
wherein each monitoring page layout setting area comprises:
a parameter setting control element, which is configured to set parameter(s) on the corresponding monitoring page;
a control element for setting a parameter display order, which is configured to set the parameter display order for the parameter(s) on the corresponding monitoring page; and
a control element for setting a parameter display area, which is configured to set the parameter display area for the parameter(s) on the corresponding monitoring page, wherein a parameter measurement result is displayed in the parameter display area.

9. The vital sign monitoring device according to claim 7, **characterized in that**, parameter(s) on the monitoring page comprise(s) direct parameter(s) which is(are) directly measured by the vital sign monitoring device, and manual parameter(s) which is(are) recorded through manual operation.

10. The vital sign monitoring device according to claim 9, **characterized in that**, the manual parameter(s) comprise(s) customized parameter(s).

11. The vital sign monitoring device according to claim 7, **characterized in that**, the parameter layout setting area comprises a locking control element, which is configured to:
lock a parameter display area to a same position on each monitoring page for display, but not lock a parameter measurement result, which is located inside the parameter display area; when switching between at least two monitoring pages.

12. The vital sign monitoring device according to claim 7, **characterized in that**, the parameter configuration setting area comprises one or more of following setting areas:
an area for setting a parameter configuration item, which is configured to receive an instruction for setting a value of the parameter configuration item;
an area for setting a monitoring mode, which is configured to receive an instruction for setting a parameter monitoring mode;
an area for setting alarm configuration, which is configured to receive an instruction for setting alarm configuration information; and
an area for setting a modular parameter monitoring tool, which is configured to receive an instruction for selecting the modular parameter monitoring tool.

13. The vital sign monitoring device according to claim 12, **characterized in that**, the area for setting the parameter configuration item comprises a control element for setting a manual parameter configuration item, which is configured to set a customized parameter configuration item.

14. The vital sign monitoring device according to claim 12, **characterized in that**, the area for setting the modular parameter monitoring tool comprises at least one of: an orthostatic hypotension evaluation tool, a multiple blood pressure measurement and recording tool, a blood pressure average measurement tool, and a cyanotic congenital heart disease monitoring tool.

15. The vital sign monitoring device according to any one of claims 1-14, **characterized in that**, the area for defining the workflow configuration information further comprises a close control element or a return control element;
when the close control element or the return control element is triggered, the area for defining the workflow configuration information is closed and the workflow configuration information is stored.

16. The vital sign monitoring device according to any one of claims 1-14, **characterized in that**, the workflow configuration information further comprises a transmission manner for parameter measurement result(s).

17. A vital sign monitoring device, **characterized in that**, comprising:
a processor, which is configured to:
in response to a working mode definition instruction, determine a working mode, wherein the working mode comprises a continuous measurement working mode and a discontinuous measurement working mode, wherein the continuous measurement working mode comprises multiple first measurement modes, and the discontinuous measurement working mode comprises multiple second measurement modes;
in response to an instruction for defining workflow configuration information, determine the workflow configuration information in the working mode, and determine a monitoring page according to the workflow configuration information;
in response to a measurement mode selection instruction, determine a target measurement mode among the multiple first measurement modes and the multiple second measurement modes; and
a human-machine interaction apparatus, which is configured to display a target monitoring page which corresponds to the target measurement mode.

18. The vital sign monitoring device according to claim 17, **characterized in that**, in order to in response to a measurement mode selection instruction, determine a target measurement mode among the multiple first measurement modes and the multiple second measurement modes, the processor is specifically configured to:
receive an identifier code which is transmitted from a code scanner, and determine a first measurement mode or a second measurement mode, which corresponds to the identifier code, as the target measurement mode;
receive biometric information which is transmitted from a biometric recognition device, and determine a first measurement mode or a second measurement mode, which corresponds to the biometric information, as the target measurement mode; and/or
receive the measurement mode selection instruction which is transmitted from another device, and determine a first measurement mode or a second measurement mode, which corresponds to the measurement mode selection instruction, as the target measurement mode.

19. The vital sign monitoring device according to claim 17, **characterized in that**, the human-machine interaction apparatus is further configured to display a monitoring situation definition area, which is configured to receive a monitoring situation definition instruction;
the processor is further configured to determine a monitoring situation according to the monitoring situation definition instruction, and determine at least some of the multiple first measurement modes and the multiple second measurement modes according to the monitoring situation.

20. A vital sign monitoring device, **characterized in that**, comprising:
a processor, which is configured to:
acquire a target configuration index;
transmit the target configuration index to a target device which is associated with the vital sign monitoring device, wherein the target device stores corresponding relationship(s) between one or more pairs of configuration index(s) and workflow configuration information, and the corresponding relationship is configured to determine target workflow configuration information which corresponds to the target configuration index; and
receive the target workflow configuration information; and
a human-machine interaction apparatus, which is configured to display a target monitoring page which corresponds to the target workflow configuration information.

21. A vital sign monitoring device, **characterized in that**, comprising:
a human-machine interaction apparatus, which is configured to receive an operation instruction, wherein the operation instruction is inputted by a device operator in a predetermined working mode and related to execution of a workflow; wherein the predetermined working mode comprises a continuous measurement working mode or a discontinuous measurement working mode; and
a processor, which is configured to acquire workflow execution information, which is generated by the vital sign monitoring device during the execution of the workflow in the predetermined working mode based on the operation instruction; and to obtain, by self-learning the workflow execution information, workflow configuration information to be recommended, which corresponds to the predetermined working mode;
wherein the human-machine interaction apparatus is further configured to display a monitoring page to be recommended, which corresponds to the recommended workflow configuration information.

22. A workflow definition method, **characterized in that**, comprising:
displaying a measurement mode identifier list, a working mode definition area, and an area for defining workflow configuration information; wherein the measurement mode identifier list comprises multiple first measurement modes and multiple second measurement modes; the measurement mode identifier list is configured to receive an measurement mode selection instruction, the working mode definition area is configured to receive a working mode definition instruction; the area for defining the workflow configuration information is configured to receive an instruction for defining the workflow configuration information;
determining a working mode according to the working mode definition instruction; wherein the working mode comprises a continuous measurement working mode and a discontinuous measurement working mode, the continuous measurement working mode comprises the multiple first measurement modes, and the discontinuous measurement working mode comprises the multiple second measurement modes;
determining workflow configuration information in the working mode according to the instruction for defining the workflow configuration information, and determining a monitoring page according to the workflow configuration information;
selecting a target measurement mode, which corresponds to target workflow configuration information, from the measurement mode identifier list, according to the measurement mode selection instruction; and
displaying a target monitoring page which corresponds to the target measurement mode.

23. The method according to claim 22, **characterized in that**, further comprising:
determining environment information of a vital sign monitoring device, and determining a monitoring situation according to the environment information; wherein the environment information comprises one or more of: voice data, text data, image data, and device position;
wherein, determining workflow configuration information in the working mode according to the instruction for defining the workflow configuration information, comprises:
determining the workflow configuration information in the working mode and the monitoring situation, according to the instruction for defining the workflow configuration information.

24. The method according to claim 22, **characterized in that**, further comprising:
displaying a monitoring situation definition area, which is configured to receive a monitoring situation definition instruction; and
determining a monitoring situation according to the monitoring situation definition instruction;
wherein, determining workflow configuration information in the working mode according to the instruction for defining the workflow configuration information, comprises:
determining the workflow configuration information in the working mode and the monitoring situation, according to the instruction for defining the workflow configuration information.

25. The method according to claim 24, **characterized in that**, the monitoring situation definition area comprises a department type definition area, which is configured to define a department type; the monitoring situation comprises the department type; the department type comprises at least one of: General Ward, Emergency Department, Doctor Office, Long Term Care, Ambulatory Surgery Center, Community Health Service Center, and Neonatal Department.

26. The method according to claim 25, **characterized in that**, the department type is classified according to national regions as follows:
when the national region is the United States, the department type comprises: General Ward, Emergency Department, Doctor Office, Long Term Care, and Ambulatory Surgery Center;
when the national region is Europe, the department type comprises: General Ward, Emergency Department, Doctor Office, and Ambulatory Surgery Center;
when the national region is China, the department type comprises: General Ward, Emergency Department, Community Health Service Center, Ambulatory Surgery Center, and Neonatal Department.

27. The method according to claim 23 or 24, **characterized in that**, the working mode definition area comprise at least one of follow scenarios:
when the monitoring situation is General Ward, the continuous measurement working mode comprises a continuous monitoring mode, while the discontinuous measurement working mode comprises a general round mode and an initial evaluation mode;
when the monitoring situation is emergency department, the continuous measurement working mode comprises a continuous monitoring mode, while the discontinuous measurement working mode comprises a triage mode and a spot check mode;
when the monitoring situation is Doctor Office or Community Health Service Center, the continuous measurement working mode comprises a continuous monitoring mode, while the discontinuous measurement working mode comprises a spot check mode;
when the monitoring situation is Long Term Care, the continuous measurement working mode comprises a continuous monitoring mode, while the discontinuous measurement working mode comprises a general round mode and an initial evaluation mode;
when the monitoring situation is Ambulatory Surgery Center, the continuous measurement working mode comprises a continuous monitoring mode, while the discontinuous measurement working mode comprises a recovery mode; and
when the monitoring situation is Neonatal Department, the continuous measurement working mode comprises a continuous monitoring mode, while the discontinuous measurement working mode comprises a spot check mode.

28. The method according to claim 22, **characterized in that**, the area for defining the workflow configuration information comprises one or more of following setting areas: a workflow name setting area, a parameter layout setting area, and a parameter configuration setting area;
wherein the method further comprises:
setting a workflow name setting instruction, based on an input instruction of the workflow name setting area;
setting a parameter type and/or a parameter display layout rule, on the monitoring page, based on an input instruction of the parameter layout setting area; and
setting at least one of followings on the monitoring page: a parameter signal acquisition way, a parameter signal processing way and a parameter measurement result display way, based on an input instruction of the parameter layout setting area.

29. The method according to claim 28, **characterized in that**, the monitoring page comprises at least two monitoring pages; the parameter layout setting area comprises at least two monitoring page layout setting areas, which are configured to correspondingly define the at least two monitoring pages; wherein each monitoring page layout setting area is configured to set the parameter type and/or the parameter display layout rule on one corresponding monitoring page;
wherein each monitoring page layout setting area comprises: a parameter setting control element, a control element for setting a parameter display order, and a control element for setting a parameter display area;
wherein the method further comprises:
setting parameter(s) on the corresponding monitoring page, based on an input instruction of the parameter setting control element;
setting the parameter display order for the parameter(s) on the corresponding monitoring page, based on an input instruction of the control element for setting the parameter display order; and
setting the parameter display area for the parameter(s) on the corresponding monitoring page, based on an input instruction of the control element for setting the parameter display area; wherein a parameter measurement result is displayed in the parameter display area.

30. The method according to claim 28, **characterized in that**, parameter(s) on the monitoring page comprise(s) direct parameter(s) which is(are) directly measured by a vital sign monitoring device and manual parameter(s) which is(are) recorded through manual operation.

31. The method according to claim 30, **characterized in that**, the manual parameter(s) comprise(s) customized parameter(s).

32. The method according to claim 28, **characterized in that**, the parameter layout setting area further comprises a locking control element;
wherein the method further comprises:
in response to a trigger operation on the locking control element, locking a parameter display area to a same position on each monitoring page for display, but not locking a parameter measurement result, which is located inside the parameter display area; when switching between at least two monitoring pages.

33. The method according to claim 28, **characterized in that**, the parameter configuration setting area comprises one or more of following setting areas: an area for setting a parameter configuration item, an area for setting a monitoring mode, an area for setting alarm configuration, and an area for setting a modular parameter monitoring tool;
wherein the method further comprises:
receiving an instruction for setting a value of the parameter configuration item, based on the area for setting the parameter configuration item;
receiving an instruction for setting a parameter monitoring mode, based on the area for setting the monitoring mode;
receiving an instruction for setting alarm configuration information, based on the area for setting the alarm configuration; and
receiving an instruction for selecting the modular parameter monitoring tool, based on the area for setting the modular parameter monitoring tool.

34. The method according to claim 33, **characterized in that**, the area for setting the parameter configuration item comprises a control element for setting a manual parameter configuration item, which is configured to set a customized parameter configuration item.

35. The method according to claim 33, **characterized in that**, the area for setting the modular parameter monitoring tool comprises at least one of: an orthostatic hypotension evaluation tool, a multiple blood pressure measurement and recording tool, a blood pressure average measurement tool, and a cyanotic congenital heart disease monitoring tool.

36. The method according to any one of claims 22-35, **characterized in that**, the area for defining the workflow configuration information further comprises a close control element or a return control element;
wherein the method further comprises:
closing the area for defining the workflow configuration information and storing the workflow configuration information when the close control element or the return control element is triggered.

37. The method according to any one of claims 22-35, **characterized in that**, the workflow configuration information further comprises a transmission manner for parameter measurement result(s).

38. A workflow definition method, **characterized in that**, comprising:
in response to a working mode definition instruction, determining a working mode, wherein the working mode comprises a continuous measurement working mode and a discontinuous measurement working mode, wherein the continuous measurement working mode comprises multiple first measurement modes, and the discontinuous measurement working mode comprises multiple second measurement modes;
in response to an instruction for defining workflow configuration information, determining the workflow configuration information in the working mode, and determining a monitoring page according to the workflow configuration information;
in response to a measurement mode selection instruction, determining a target measurement mode among the multiple first measurement modes and the multiple second measurement modes; and
displaying a target monitoring page which corresponds to the target measurement mode.

39. The method according to claim 38, **characterized in that**, in response to a measurement mode selection instruction, determining a target measurement mode among the multiple first measurement modes and the multiple second measurement modes, comprises
receiving an identifier code which is transmitted from a code scanner, and determining a first measurement mode or a second measurement mode, which corresponds to the identifier code, as the target measurement mode;
receiving biometric information which is transmitted from a biometric recognition device, and determining a first measurement mode or a second measurement mode, which corresponds to the biometric information, as the target measurement mode; and/or
receiving the measurement mode selection instruction which is transmitted from another device, and determining a first measurement mode or a second measurement mode, which corresponds to the measurement mode selection instruction, as the target measurement mode.

40. The method according to claim 38, **characterized in that**, further comprising:
displaying a monitoring situation definition area, which is configured to receive a monitoring situation definition instruction;
determining a monitoring situation according to the monitoring situation definition instruction; and
determining at least some of the multiple first measurement modes and the multiple second measurement modes according to the monitoring situation.

41. A workflow definition method, **characterized in that**, comprising:
acquiring a target configuration index;
transmitting the target configuration index to a target device, wherein, the target device stores corresponding relationship(s) between one or more pairs of configuration index(s) and workflow configuration information, and the corresponding relationship is configured to determine target workflow configuration information which corresponds to the target configuration index;
receiving the target workflow configuration information; and
displaying a target monitoring page which corresponds to the target workflow configuration information.

42. A workflow definition method, **characterized in that**, comprising:
receiving an operation instruction, which is inputted by a device operator in a predetermined working mode and related to execution of a workflow, wherein the predetermined working mode comprises a continuous measurement working mode or a discontinuous measurement working mode;
acquiring workflow execution information, which is generated by a vital sign monitoring device during the execution of the workflow in the predetermined working mode based on the operation instruction;
obtaining, by self-learning the workflow execution information, workflow configuration information to be recommended, which corresponds to the predetermined working mode; and
displaying a monitoring page to be recommended, which corresponds to the recommended workflow configuration information.
